(19) Europäisches Patentamt
European Patent Office
Office européen des brevets

(11) Veröffentlichungsnummer: **0 384 192 A2**

(12) **EUROPÄISCHE PATENTANMELDUNG**

(21) Anmeldenummer: **90102138.6**

(22) Anmeldetag: **03.02.90**

(51) Int. Cl.[5]: **C07D 209/48, C07D 209/46, C07D 209/44, C07D 471/04, C07C 217/84, C07C 43/12, C07C 43/13, C07C 213/06, C07C 213/02, A01N 37/32, //(C07D471/04,235:00,221:00)**

(30) Priorität: **18.02.89 DE 3905006**

(43) Veröffentlichungstag der Anmeldung: **29.08.90 Patentblatt 90/35**

(84) Benannte Vertragsstaaten: **BE CH DE DK FR GB IT LI NL**

(71) Anmelder: **BAYER AG**

**D-5090 Leverkusen 1 Bayerwerk(DE)**

(72) Erfinder: **Schallner, Otto, Dr.**
**Noldeweg 22**
**D-4019 Monheim(DE)**
Erfinder: **Negele, Michael, Dr.**
**Wolfskaul 6**
**D-5000 Köln 80(DE)**
Erfinder: **Santel, Hans-Joachim**
**Grünstrasse 9a**
**D-5090 Leverkusen 1(DE)**
Erfinder: **Lürssen, Klaus, Dr.**
**August-Kierspel-Strasse 145**
**D-5060 Bergisch-Gladbach 2(DE)**
Erfinder: **Schmidt, Robert, Dr.**
**Im Waldwinkel 110**
**D-5060 Bergisch-Gladbach 2(DE)**
Erfinder: **Krauskopf, Birgit**
**Kicke 19**
**D-5060 Bergisch-Gladbach 1(DE)**

(54) **N-Aryl-Stickstoffheterocyclen mit fluorhaltigen Substituenten.**

(57) Die Erfindung betrifft neue N-Aryl-Stickstoffheterocyclen mit fluorhaltigen Substituenten der Formel (I)

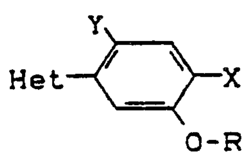

(I)

in welcher
Het für eine der nachstehenden heterocyclischen Gruppierungen

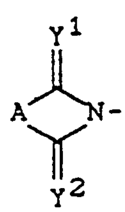

oder

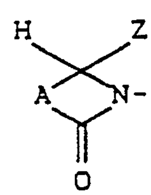

EP 0 384 192 A2

steht,
wobei
A für eine der nachstehenden Gruppierungen steht,

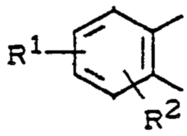 , 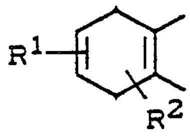 , 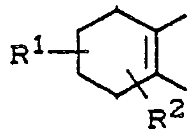 , 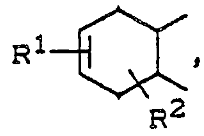 ,

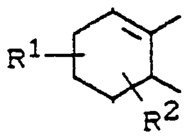 , 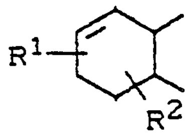 , 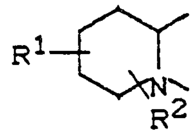 , 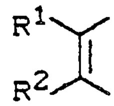 ,

R für jeweils gegebenenfalls verzweigtes, jeweils durch wenigstens ein Sauerstoffatom unterbrochenes und jeweils durch wenigstens ein Fluoratom substituiertes Alkyl, Alkenyl, Cycloalkyl, Cycloalkenyl, Cycloalkylalkyl oder Cycloalkenylalkyl steht,
X für Wasserstoff oder Halogen steht und
Y für Wasserstoff oder Halogen steht,
und die übrigen Substituenten die in der Beschreibung angegebene Bedeutung haben, mehrere Verfahren sowie neue Zwischenprodukte zu ihrer Herstellung und ihre Verwendung als Herbizide und Pflanzenwuchsregulatoren.

# N-Aryl-Stickstoffheterocyclen mit fluorhaltigen Substituenten

Die vorliegende Erfindung betrifft neue N-Aryl-Stickstoffheterocyclen mit fluorhaltigen Substituenten, Verfahren zu ihrer Herstellung und ihre Verwendung als Herbizide und als Pflanzenwuchsregulatoren sowie neue Zwischenprodukte.

Es ist bekannt, daß bestimmte Stickstoffheterocyclen, die z. B. 5-tert-Butyl-3-(2,4-dichlor-5-isopropoxyphenyl)-1,3,4-oxadiazol-2-on (Oxadiazone/®Ronstar), herbizide Eigenschaften aufweisen (vgl. US-P 3 835 862).

Die Wirkung dieser Verbindung ist jedoch bei niedrigen Aufwandmengen bzw. Wirkstoffkonzentrationen unbefriedigend.

Es wurden nun die neuen N-Aryl-Stickstoffheterocyclen mit fluorhaltigen Substituenten der allgemeinen Formel (I)

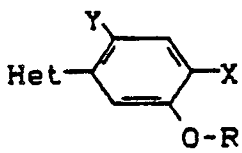

(I)

in welcher

Het für eine der nachstehenden heterocyclischen Gruppierungen

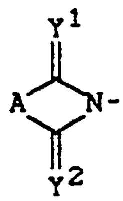 oder 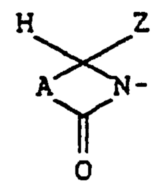

steht,

wobei

A für eine der nachstehenden Gruppierungen steht,

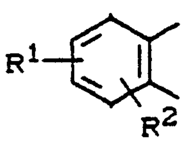, 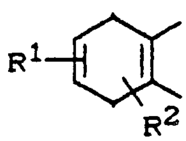, 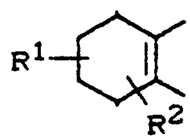, 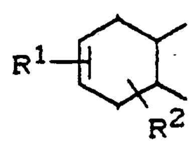,

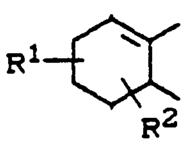, 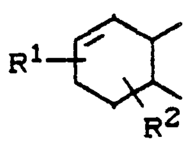, 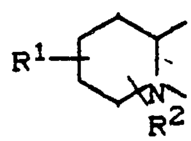, 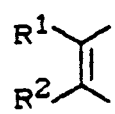,

wobei

$R^1$ und $R^2$ jeweils unabhängig voneinander für Wasserstoff, Halogen, Halogenalkyl oder Alkyl stehen,

$Y^1$ und $Y^2$ jeweils für Sauerstoff oder Schwefel stehen und

Z für Wasserstoff, Hydroxy oder Chlor steht,

R für jeweils gegebenenfalls verzweigtes, jeweils durch wenigstens ein Sauerstoffatom unterbrochenes und jeweils durch wenigstens ein Fluoratom substituiertes Alkyl, Alkenyl, Cycloalkyl, Cycloalkenyl, Cycloalkylalkyl oder Cycloalkenylalkyl steht,

X für Wasserstoff oder Halogen steht und

Y für Wasserstoff oder Halogen steht,

gefunden.

Weiter wurde gefunden, daß man die neuen N-Aryl-Stickstoffheterocyclen mit fluorhaltigen Substituenten der allgemeinen Formel (I) erhält, wenn man

(a) für den Fall, daß Het für die Gruppierung

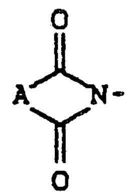

steht und
A, R, X und Y die oben angegebenen Bedeutungen haben,
cyclische Anhydride der allgemeinen Formel (II)

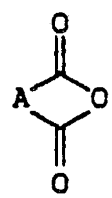

(II)

in welcher
A die oben angegebene Bedeutung hat,
mit Arylaminen der allgemeinen Formel (III)

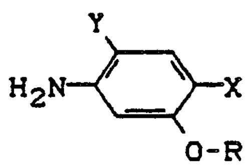

(III)

in welcher
R, X und Y die oben angegebenen Bedeutungen haben,
gegebenenfalls in Gegenwart eines Verdünnungsmittels und gegebenenfalls in Gegenwart eines Reaktionshilfsmittels umsetzt, oder wenn man

(b) für den Fall, daß Het für die Gruppierung

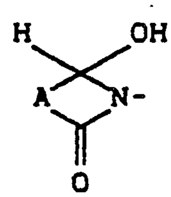

steht und
A, R, X und Y die oben angegebenen Bedeutungen haben,
substituierte Arylimide der allgemeinen Formel (Ia)

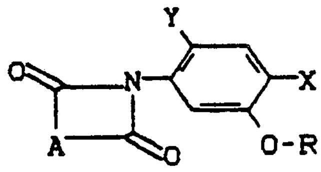

(Ia)

in welcher
A, R, X und Y die oben angegebenen Bedeutungen haben,
mit einem Reduktionsmittel, gegebenenfalls in Gegenwart eines Verdünnungsmittels umsetzt, oder wenn man

(c) für den Fall, daß Het für die Gruppierung

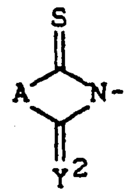

steht und
A, R, X, Y und $Y^2$ die oben angegebenen Bedeutungen haben,
substituierte Arylimide der allgemeinen Formel (Ia)

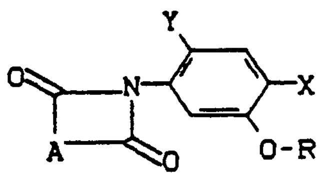

(Ia)

in welcher
A, R, X und Y die oben angegebenen Bedeutungen hahen,
mit einem Schwefelungsmittel, gegebenenfalls in Gegenwart eines Verdünnungsmittel umsetzt, oder wenn man

(d) für den Fall, daß Het für die Gruppierung

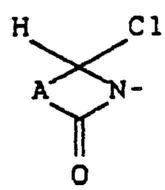

steht und
A, R, X und Y die oben angegebenen Bedeutungen haben,
N-Aryl-Stickstoffheterocyclen der allgemeinen Formel (Ib)

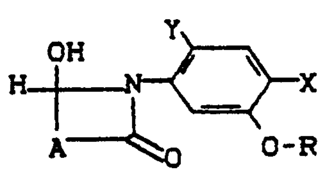

(Ib)

in welcher
A, R, X und Y die oben angegebenen Bedeutungen haben,

mit Thionylchlorid, gegebenenfalls in Gegenwart eines Verdünnungsmittels und gegebenenfalls in Gegenwart eines Reaktionshilfsmittels umsetzt, oder wenn man

(e) für den Fall, daß Het für die Gruppierung

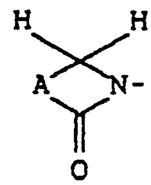

steht und

A, R, X und Y die oben angegebenen Bedeutungen haben,

N-Aryl-Stickstoffheterocyclen der allgemeinen Formel (Ic)

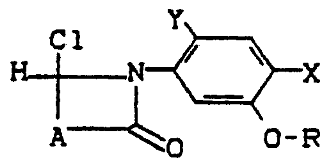

(Ic)

in welcher

A, R, X und Y die oben angegebenen Bedeutungen haben,

mit Wasserstoff, in Gegenwart eines Katalysators sowie gegebenenfalls in Gegenwart eines Verdünnungsmittels und gegebenenfalls in Gegenwart eines Säurebindemittels umsetzt, oder wenn man

(f) für den Fall, daß Het für die Gruppierung

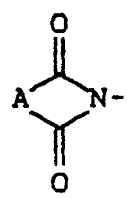

steht und

A, R, X und Y die oben angegebenen Bedeutungen haben,

Hydroxyarylimide der allgemeinen Formel (IV)

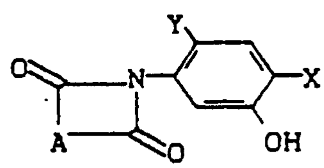

(IV)

in welcher

A, X und Y die oben angegebenen Bedeutungen haben,

mit Alkylierungsmitteln der allgemeinen Formel (V)

$X^1 - R$ (V)

in welcher

R die oben angegebene Bedeutung hat und

$X^1$ für eine nucleophile Abgangsgruppe steht,

gegebenenfalls in Gegenwart eines Säureakzeptors und gegebenenfalls in Gegenwart eines Verdünnungsmittels umsetzt, oder wenn man

(g) für den Fall, daß Het für die Gruppierung

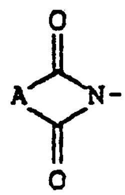

steht und
A, R und Y die oben angegebenen Bedeutungen haben sowie
X für Halogen steht,
substituierte Arylimide der allgemeinen Formel (Id)

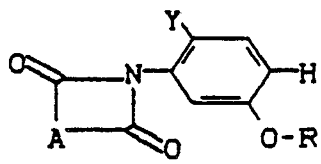

(Id)

in welcher
A, R und Y die oben angegebenen Bedeutungen haben,
mit einem Halogenierungsmittel, gegebenenfalls in Gegenwart eines Katalysators und gegebenenfalls in Gegenwart eines Verdünnungsmittels umsetzt, oder wenn man

(h) für den Fall, daß Het für die Gruppierung

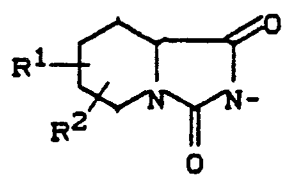

steht und
R, $R^1$, $R^2$, X und Y die oben angegebenen Bedeutungen haben,
Arylamine der allgemeinen Formel (III)

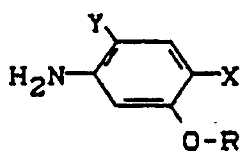

(III)

in welcher
R, X und Y die oben angegebenen Bedeutungen haben,
mit Chlorameisensäureestern der allgemeinen Formel (VI)

$R^3O - CO - Cl$ (VI)

in welcher
$R^3$ für $C_1$-$C_4$-Alkyl, Benzyl oder Phenyl steht,
gegebenenfalls in Gegenwart eines Säureakzeptors und gegebenenfalls in Gegenwart eines Verdünnungsmittels umsetzt und die hierbei gebildeten
Arylurethane der allgemeinen Formel (VII)

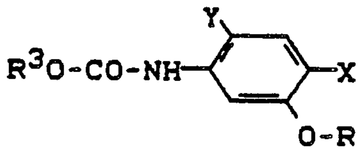 (VII)

in welcher
R, $R^3$, X und Y die oben angegebenen Bedeutungen haben,
mit Piperidin-2-carbonsäureestern der allgemeinen Formel (VIII)

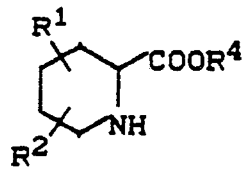 (VIII)

in welcher
$R^1$ und $R^2$ die oben angegebenen Bedeutungen haben und
$R^4$ für $C_1$-$C_4$-Alkyl steht,
gegebenenfalls in Gegenwart eines Verdünnungsmittels umsetzt.

Schließlich wurde gefunden, daß die neuen N-Aryl-Stickstoffheterocyclen mit fluorhaltigen Substituenten der allgemeinen Formel (I) herbizide und pflanzenwuchsregulierende Eigenschaften aufweisen.

Überraschenderweise sind die erfindungsgemäßen N-Aryl-Stickstoffheterocyclen mit fluorhaltigen Substituenten der Formel (I) gegen Unkräuter wesentlich stärker wirksam als 5-tert-Butyl-3-(2,4-dichlor-5-isopropoxy-phenyl)-1,3,4-oxadiazol-2-on, welches ein strukturell ähnlicher vorbekannter Wirkstoff gleicher Wirkungsrichtung ist.

Die Erfindung betrifft vorzugsweise Verbindungen der Formel (I), in welcher
Het für eine der nachstehenden heterocyclischen Gruppierungen

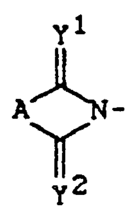 oder 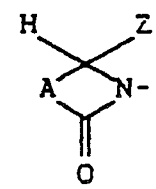

steht,
wobei
A für eine der nachstehenden Gruppierungen steht,

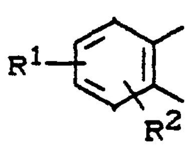 , 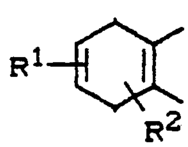 , 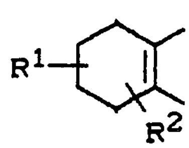 , 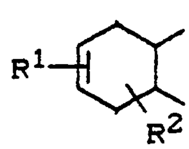 ,

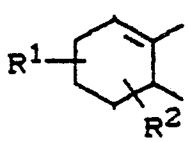 , 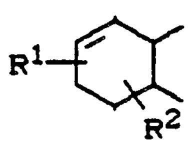 , 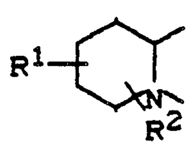 , 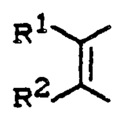 ,

wobei
$R^1$ und $R^2$ jeweils unabhängig voneinander für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit 1 bis 5 gleichen oder verschiedenen Halogenatomen, insbesondere Fluor oder Chlor,

stehen,
$Y^1$ und $Y^2$ für Sauerstoff oder Schwefel stehen,
Z für Wasserstoff, Hydroxy oder Chlor steht,
R für jeweils gegebenenfalls verzweigtes, jeweils durch 1 bis 4 Sauerstoffatome unterbrochenes und jeweils durch 1 bis 5 Fluoratome substituiertes Alkyl, Alkenyl, Cycloalkyl, Cycloalkenyl, Cycloalkylalkyl oder Cycloalkenylalkyl mit jeweils bis zu 20, vorzugsweise bis zu 15, insbesondere bis zu 10 Kohlenstoffatomen steht,
X für Wasserstoff, Fluor, Chlor oder Brom steht und
Y für Wasserstoff, Fluor oder Chlor steht.

Die Erfindung betrifft insbesondere Verbindungen der Formel (I), in welcher
Het für eine der nachstehenden heterocyclischen Gruppierungen

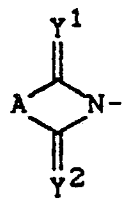 oder 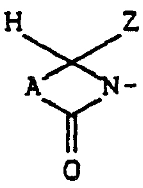

steht,
wobei
A für eine der nachstehenden Gruppierungen steht,

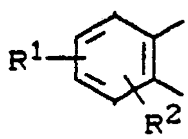, 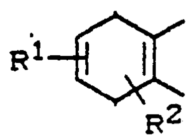, 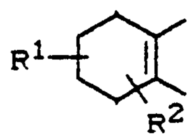, 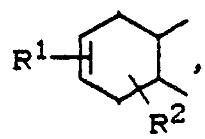,

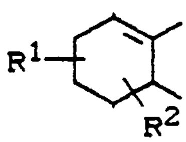, 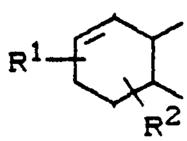, 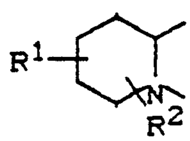, 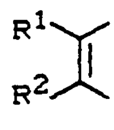,

wobei
$R^1$ und $R^2$ jeweils unabhängig voneinander für Wasserstoff, Methyl oder Trifluormethyl stehen,
$Y^1$ und $Y^2$ für Sauerstoff oder Schwefel stehen,
Z für Wasserstoff, Hydroxy oder Chlor steht,
R für jeweils gegebenenfalls verzweigtes und jeweils durch 2 bis 4 Fluoratome substituiertes Oxaalkyl oder Dioxaalkyl mit jeweils bis zu 10, insbesondere bis zu 5, 6, 7 oder 8 Kohlenstoffatomen steht,
X für Wasserstoff, Chlor oder Brom steht und
Y für Wasserstoff oder Fluor steht.

In den aufgezählten Definitionsbereichen sind ganz besonders die folgenden Heterocyclen bevorzugt:

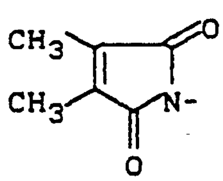 oder 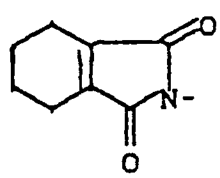

Beispiele für die erfindungsgemäßen Verbindungen der Formel (I) sind in der nachstehenden Tabelle 1 aufgeführt - vgl. auch die Herstellungsbeispiele.

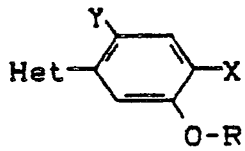

(I)

Tabelle 1: Beispiele für die Verbindungen der Formel (I)

| Het | X | Y | R |
|---|---|---|---|
| 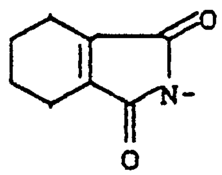 | H | H | $-CH_2CH_2-O-CH_2CH_2-O-CHF_2$ |
| 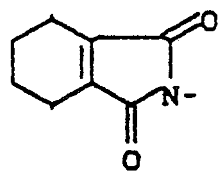 | H | H | $-CH_2CH_2-O-CH_2CH_2-O-CH_2CF_3$ |
| 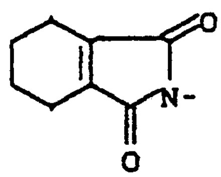 | F | F | $-CH_2CH_2-O-CH_2CH_2-O-CHF_2$ |
| 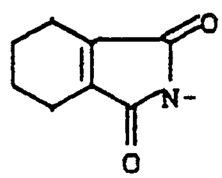 | F | F | $-CH_2CH_2-O-CH_2CH_2-O-CH_2CF_3$ |

Tabelle 1 - Fortsetzung

| Het | X | Y | R |
|---|---|---|---|
| 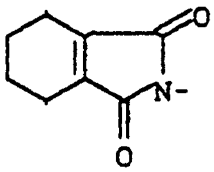 | Cl | H | $-CH_2CH_2-O-CH_2CH_2-O-CHF_2$ |
| 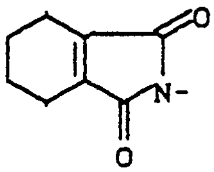 | Cl | F | $-CH_2CH_2-O-CH_2CH_2-O-CH_2CF_3$ |
| 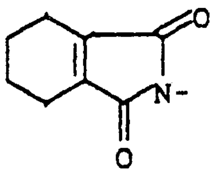 | Br | H | $-CH_2CH_2-O-CH_2CH_2-O-CHF_2$ |
| 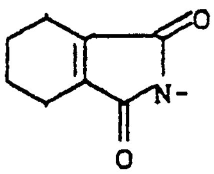 | Br | F | $-CH_2CH_2-O-CH_2CH_2-O-CH_2CF_3$ |
| 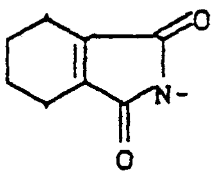 | Cl | H | $-CH_2CH_2-O-CH_2CF_3$ |
| 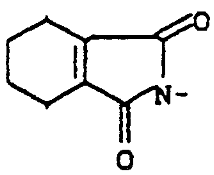 | Cl | F | $-CH_2CH_2-O-CH_2CF_3$ |
| 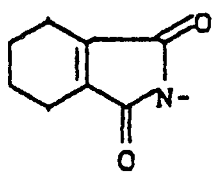 | Cl | F | $-CH_2CH_2-O-CH_2-CH(CH_2F)_2$ |

Tabelle 1 - Fortsetzung

| Het | X | Y | R |
|---|---|---|---|
| 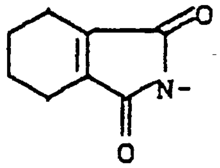 | Cl | F | $-CHCH_2-O-CH_2CF_3$<br>$CH_3$ |
| 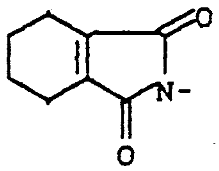 | Cl | F | $-CHCH_2-O-CH_2CF_3$<br>$C_2H_5$ |
| 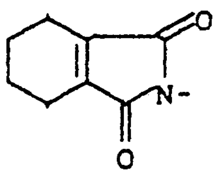 | Cl | F | $-CHCH_2-O-CH_2CF_3$<br>$CH_2F$ |
| 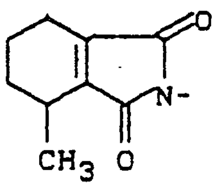 | CL | F | $-CH_2CH_2-O-CH_2CH_2-O-CHF_2$ |
| 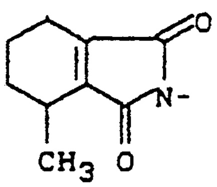 | F | F | $-CH_2CH_2-O-CH_2CH_2-O-CH_2CF_3$ |
| 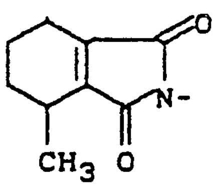 | Br | F | $-CH_2CH_2-O-CH_2CH_2-O-CHF_2$ |
| 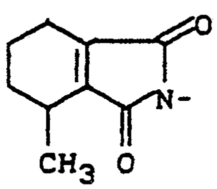 | Cl | F | $-CH_2CH_2-O-CH_2CF_3$ |

Tabelle 1 - Fortsetzung

| Het | X | Y | R |
|---|---|---|---|
| 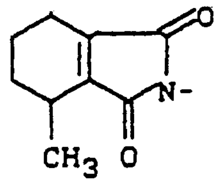 | Cl | F | $-CH_2CH_2-O-CHCF_2$ |
| 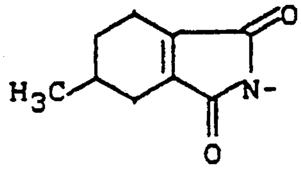 | F | F | $-CH_2CH_2-O-CH_2CH_2-O-CHF_2$ |
| 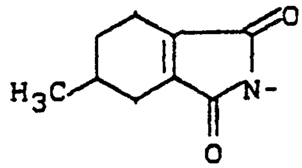 | H | H | $-CH_2CH_2-O-CH_2CH_2-O-CH_2CF_3$ |
| 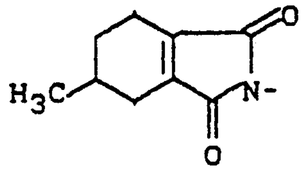 | Cl | F | $-CH(CH_3)CH_2-O-CH_2CF_3$ |
| 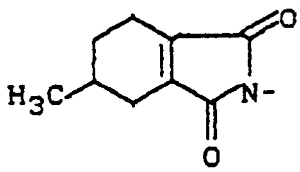 | F | F | $-CH(CH_2F)CH_2-O-CH_2CF_3$ |
| 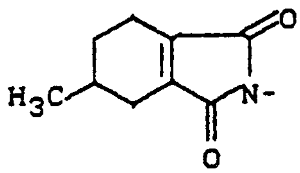 | Cl | F | $-CH_2CH_2-O-CH_2CH_2-O-CH_2CF_3$ |
| 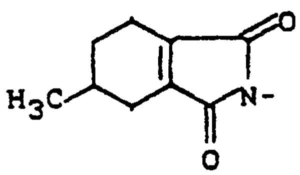 | Cl | F | $-CH_2CH_2-O-CH_2CF_3$ |

Tabelle 1 - Fortsetzung

| Het | X | Y | R |
| --- | --- | --- | --- |
| 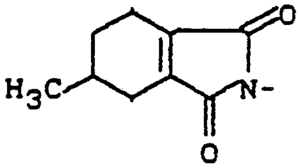 | Cl | F | $-CH_2CH_2-O-CHF_2$ |
| 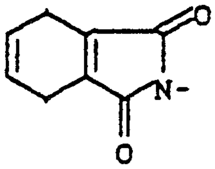 | F | F | $-CH_2CH_2-O-CH_2CH_2-O-CH_2CF_3$ |
| 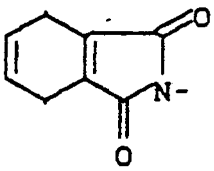 | H | H | $-CH_2CH_2-O-CH_2CH_2-O-CH_2CF_3$ |
| 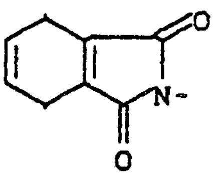 | Br | F | $-CH_2CH_2-O-CH_2CH_2-O-CH_2CF_3$ |
| 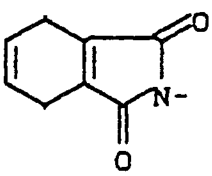 | Cl | F | $-CH_2CH_2-O-CH_2CH_2-O-CHF_2$ |
| 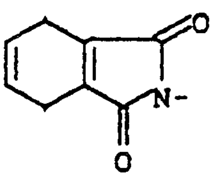 | Cl | F | $-\underset{\underset{CH_3}{\vert}}{C}HCH_2-O-CH_2CF_3$ |
| 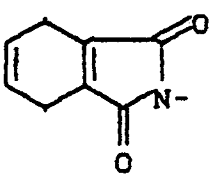 | Cl | F | $-CH_2CH_2-O-CH_2CF_3$ |

Tabelle 1 - Fortsetzung

| Het | X | Y | R |
|---|---|---|---|
| 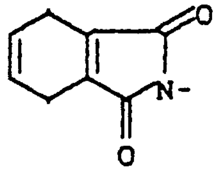 | Cl | F | $-CHCH_2-O-CHF_2$<br>\|<br>$CH_3$ |
| 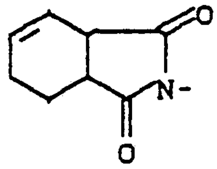 | F | F | $-CH_2CH_2-O-CH_2CH_2-O-CHF_2$ |
| 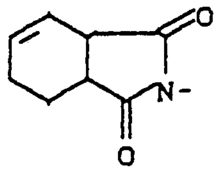 | H | H | $-CH_2CH_2-O-CH_2CH_2-O-CH_2CF_3$ |
| 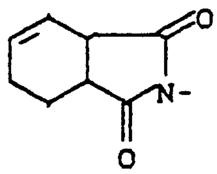 | Cl | F | $-CHCH_2-O-CH_2CF_3$<br>\|<br>$CH_2F$ |
| 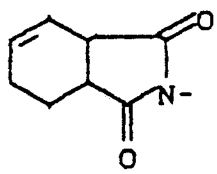 | Cl | F | $-CH_2CH_2-O-CH_2CH_2-O-CH_2CF_3$ |
| 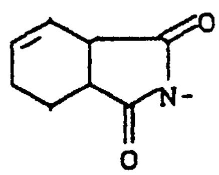 | Cl | F | $-CHCH_2-O-CH_2CF_3$<br>\|<br>$C_2H_5$ |
| 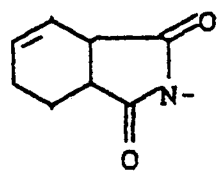 | Cl | F | $-CH_2CH_2-O-CH_2CF_3$ |

Tabelle 1 - Fortsetzung

| Het | X | Y | R |
|---|---|---|---|
| 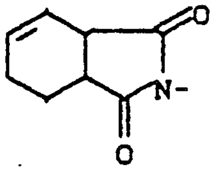 | Cl | F | $-CH_2CH_2-O-CHF_2$ |
| 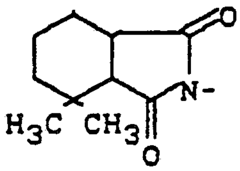 | F | F | $-CH_2CH_2-O-CH_2CH_2-O-CHF_2$ |
| 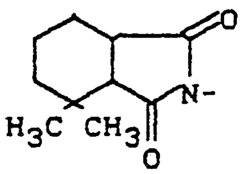 | H | H | $-CH_2CH_2-O-CH_2CH_2-O-CH_2CF_3$ |
| 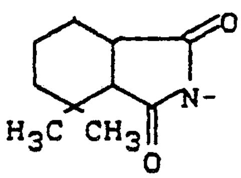 | Cl | F | $-CH(CH_3)CH_2-O-CH_2CH_2-O-CHF_2$ |
| 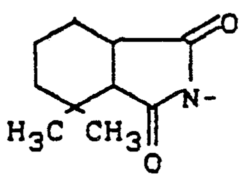 | Cl | F | $-CH_2CH_2-O-CH_2CH_2-O-CH_2CF_3$ |
| 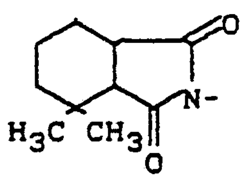 | Br | F | $-CH_2CH_2-O-CH_2CH_2-O-CHF_2$ |
| 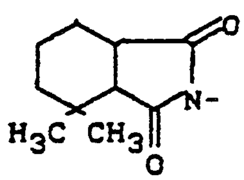 | Cl | F | $-CH_2CH_2-O-CH_2CF_3$ |

Tabelle 1 - Fortsetzung

| Het | X | Y | R |
|---|---|---|---|
| 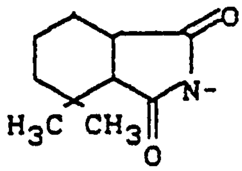 | Cl | F | $-CHCH_2-O-CH_2CF_3$<br>$CH_2F$ |
| 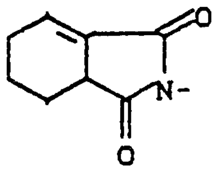 | F | F | $-CH_2CH_2-O-CH_2CH_2-O-CHF_3$ |
| 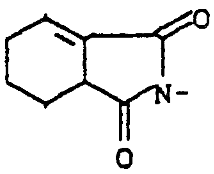 | H | H | $-CH_2CH_2-O-CH_2CH_2-O-CH_2CF_3$ |
| 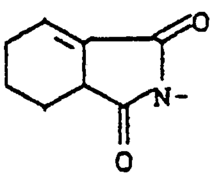 | Cl | F | $-CHCH_2-O-CH_2CF_3$<br>$CH_3$ |
| 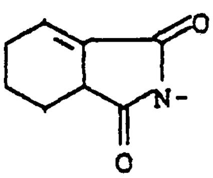 | Br | F | $-CHCH_2-O-CH_2CF_3$<br>$CH_2F$ |
| 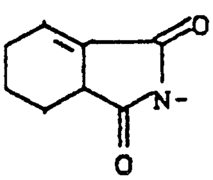 | Cl | F | $-CHCH_2-O-CH_2CF_3$<br>$C_2H_5$ |
| 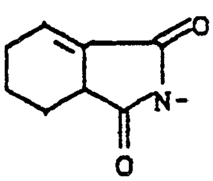 | Cl | F | $-CH_2CH_2-O-CH_2CF_3$ |

Tabelle 1 - Fortsetzung

| Het | X | Y | R |
|---|---|---|---|
| 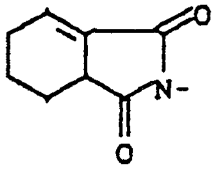 | Cl | F | $-CHCH_2-O-CHF_2$<br>$CH_3$ |
| 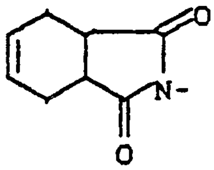 | F | F | $-CH_2CH_2-O-CH_2CH_2-O-CHF_2$ |
| 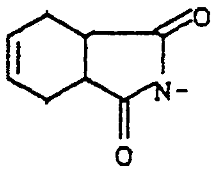 | H | H | $-CH_2CH_2-O-CH_2CH_2-O-CH_2CF_3$ |
| 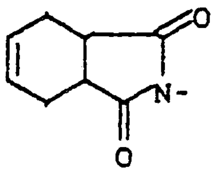 | Cl | F | $-CHCH_2-O-CH_2CF_3$<br>$CH_3$ |
| 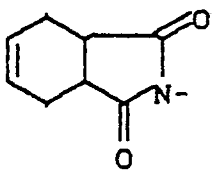 | Cl | F | $-CHCH_2-O-CH_2CF_3$<br>$CH_2F$ |
| 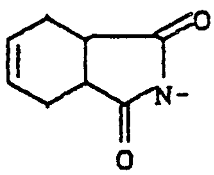 | Br | F | $-CH_2CH_2-O-CH$ $CH_2F$<br>$CH_2F$ |
| 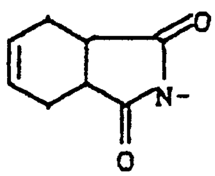 | Cl | F | $-CH_2CH_2-O-CH_2CF_3$ |

Tabelle 1 - Fortsetzung

| Het | X | Y | R |
|---|---|---|---|
| 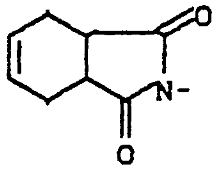 | Cl | F | $-\underset{\mid \atop CH_3}{C}HCH_2-O-CHF_2$ |
| 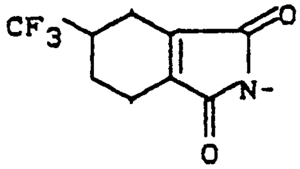 | F | F | $-CH_2CH_2-O-CH_2CH_2-O-CHF_2$ |
| 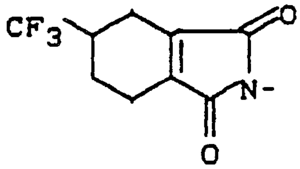 | H | H | $-CH_2CH_2-O-CH_2CH_2-O-CH_2CF_3$ |
| 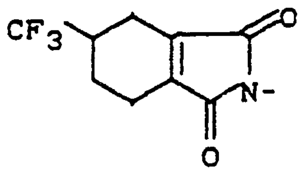 | Cl | F | $-\underset{\mid \atop CH_3}{C}HCH_2-O-CH_2CH_2-O-CHF_2$ |
| 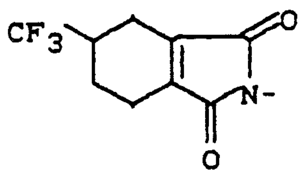 | Cl | F | $-\underset{\mid \atop CH_2F}{C}HCH_2-O-CH_2CF_3$ |
| 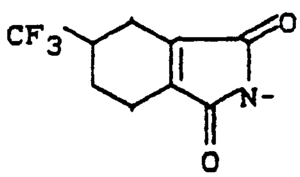 | Br | F | $-\underset{\mid \atop C_2H_5}{C}HCH_2-O-CH_2CF_3$ |
| 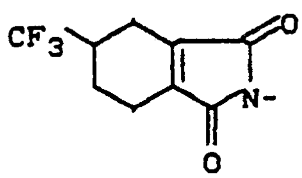 | Cl | F | $-CH_2CH_2-O-CH_2CF_3$ |

<u>Tabelle 1</u> - Fortsetzung

| Het | X | Y | R |
|---|---|---|---|
| 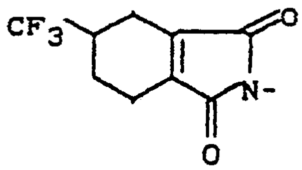 | Cl | F | $-CHCH_2-O-CHF_2$<br>$CH_3$ |
| 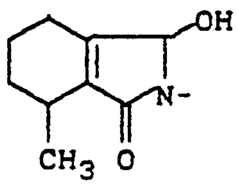 | F | F | $-CH_2CH_2-O-CH_2CH_2-O-CHF_2$ |
| 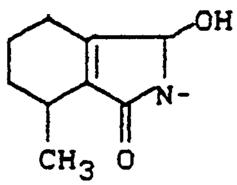 | H | H | $-CH_2CH_2-O-CH_2CH_2-O-CH_2CF_3$ |
| 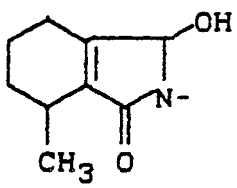 | Cl | F | $-CHCH_2-O-CH_2CH_2-O-CHF_2$<br>$CH_3$ |
| 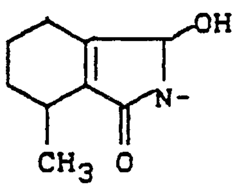 | Cl | F | $-CHCH_2-O-CH_2CF_3$<br>$CH_2F$ |
| 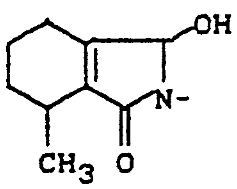 | Br | F | $CH_2F$<br>$-CH_2CH_2-O-CH$<br>$CH_2F$ |
| 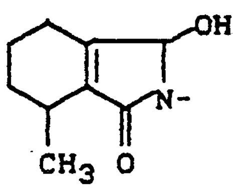 | Cl | F | $-CH_2CH_2-O-CH_2CF_3$ |

Tabelle 1 - Fortsetzung

| Het | X | Y | R |
|---|---|---|---|
| 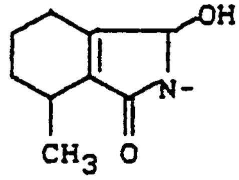 | Cl | F | 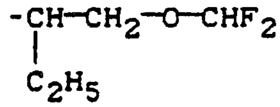 |
| 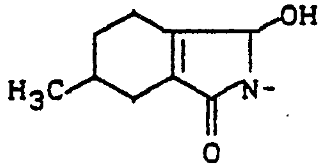 | F | F | $-CH_2CH_2-O-CH_2CH_2-O-CHF_2$ |
| 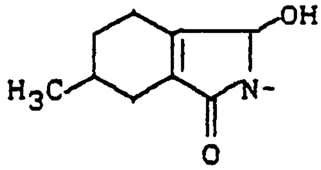 | H | H | $-CH_2CH_2-O-CH_2CH_2-O-CH_2CF_3$ |
| 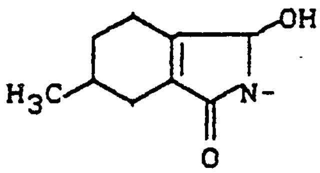 | Cl | F | 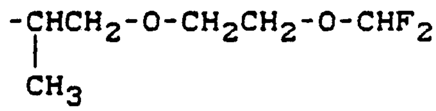 |
| 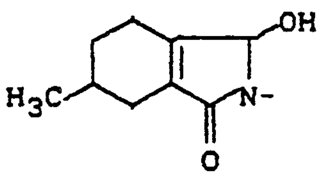 | Cl | F | 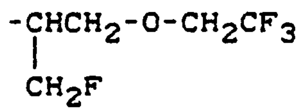 |
| 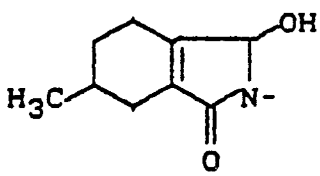 | Br | F | 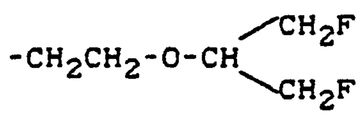 |
| 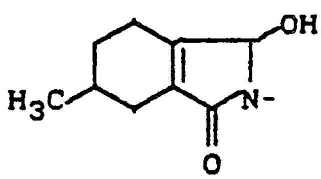 | Cl | F | $-CH_2CH_2-O-CH_2CF_3$ |

Tabelle 1 - Fortsetzung

| Het | X | Y | R |
|---|---|---|---|
| 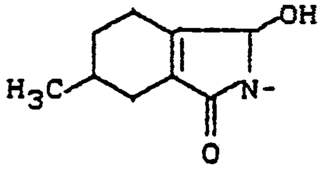 | Cl | F | $-CHCH_2-O-CH_2CF_3$<br>$CH_3$ |
| 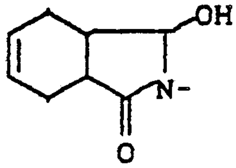 | F | F | $-CH_2CH_2-O-CH_2CH_2-O-CHF_2$ |
| 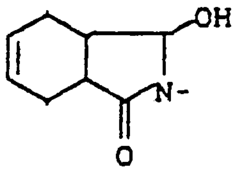 | H | H | $-CH_2CH_2-O-CH_2CH_2-O-CH_2CF_3$ |
| 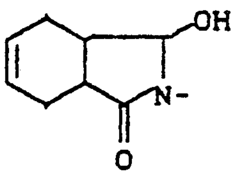 | Cl | F | $-CHCH_2-O-CH_2CH_2-O-CHF_2$<br>$CH_3$ |
| 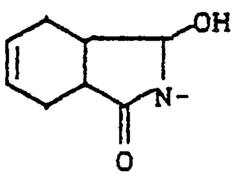 | Cl | F | $-CHCH_2-O-CH_2CF_3$<br>$CH_2F$ |
| 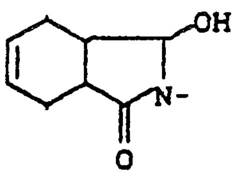 | Br | F | $CH_2F$<br>$-CH_2CH_2-O-CH$<br>$CH_2F$ |
| 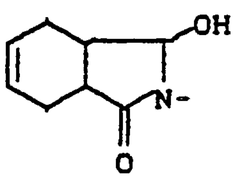 | Cl | F | $-CH_2CH_2-O-CH_2CF_3$ |

<u>Tabelle 1</u> - Fortsetzung

| Het | X | Y | R |
|---|---|---|---|
| 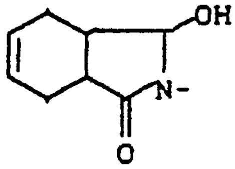 | Cl | F | $-\underset{\displaystyle CH_3}{CH}CH_2-O-CHF_2$ |
| 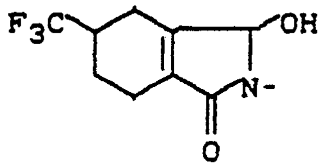 | F | F | $-CH_2CH_2-O-CH_2CH_2-O-CHF_2$ |
| 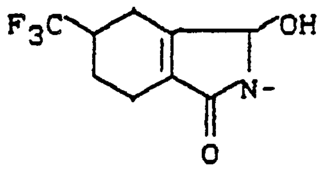 | H | H | $-CH_2CH_2-O-CH_2CH_2-O-CH_2CF_3$ |
| 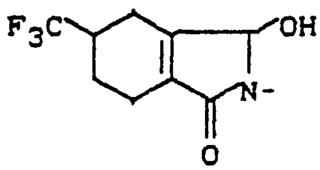 | Cl | F | $-\underset{\displaystyle CH_3}{CH}CH_2-O-CH_2CH_2-O-CHF_2$ |
| 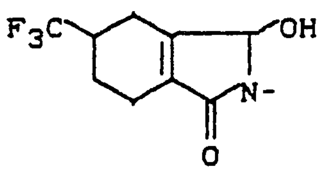 | Cl | F | $-\underset{\displaystyle C_2H_5}{CH}CH_2-O-CH_2CF_3$ |
| 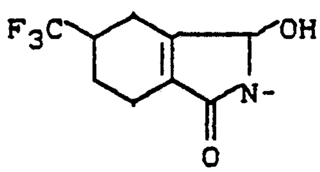 | Br | F | $-CH_2CH_2-O-CH\begin{smallmatrix}CH_2F\\CH_2F\end{smallmatrix}$ |
| 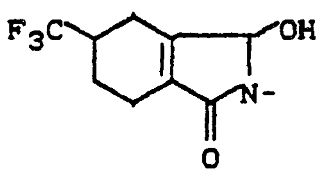 | Cl | F | $-CH_2CH_2-O-CH_2CF_3$ |

Tabelle 1 - Fortsetzung

| Het | X | Y | R |
|---|---|---|---|
| 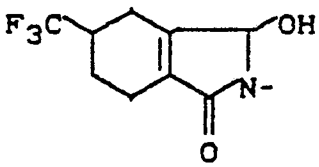 | Cl | F | $-CHCH_2-O-CHF_2$<br>$\vert$<br>$CH_3$ |
| 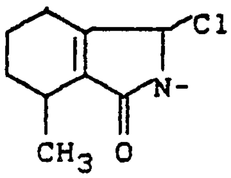 | F | F | $-CH_2CH_2-O-CH_2CH_2-O-CH_2CF_3$ |
| 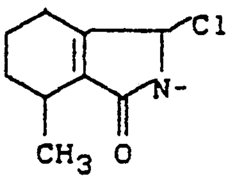 | H | H | $-CH_2CH_2-O-CH_2CH_2-O-CHF_2$ |
| 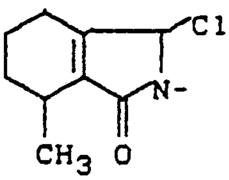 | Cl | F | $-CHCH_2-O-CH_2CH_2-O-CHF_2$<br>$\vert$<br>$CH_3$ |
| 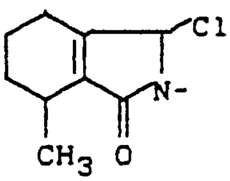 | Cl | F | $-CHCH_2-O-CH_2CF_3$<br>$\vert$<br>$CH_2F$ |
| 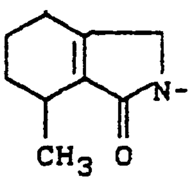 | F | F | $CH_2F$<br>$-CH_2CH_2-O-CH$<br>$CH_2F$ |
| 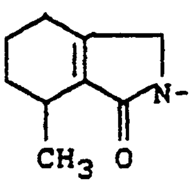 | Cl | F | $-CH_2CH_2-O-CH_2CH_2-O-CF_2CHF_2$ |

Tabelle 1 - Fortsetzung

| Het | X | Y | R |
|---|---|---|---|
| 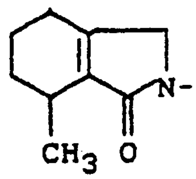 | Br | F | $-CH_2CH_2-O-CH_2CH_2-O-CHF_2$ |
| 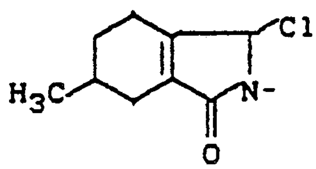 | F | F | $-CH_2CH_2-O-CH_2CH_2-O-CH_2CF_3$ |
| 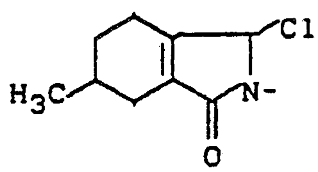 | Cl | F | $-CH_2CH_2-O-CH_2CH_2-O-CHF_2$ |
| 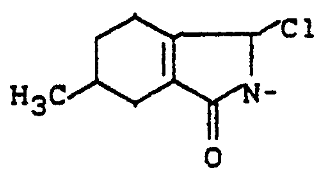 | Cl | F | $-CH_2CH_2-O-CH_2CH_2-O-CH_2CF_3$ |
| 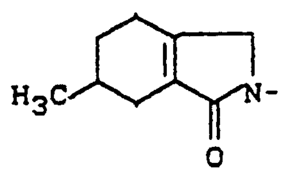 | F | F | $-CHCH_2-O-CH_2CF_3$<br>\|<br>$CH_3$ |
| 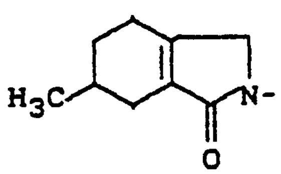 | Cl | F | $CH_2F$<br>$-CH_2CH_2-O-CH$<br>$CH_2F$ |
| 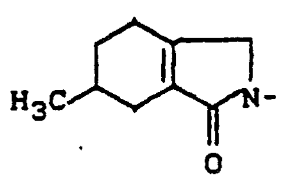 | Br | F | $-CH_2CH_2-O-CH_2CH_2-O-CHF_2$ |

Tabelle 1 - Fortsetzung

| Het | X | Y | R |
|---|---|---|---|
| 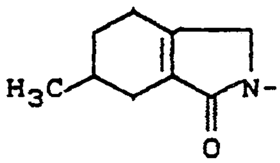 | Cl | F | $-CH_2CH_2-O-CH_2CF_3$ |
| 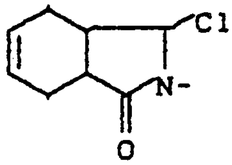 | F | F | $-CH_2CH_2-O-CH_2CH_2-O-CH_2CF_3$ |
| 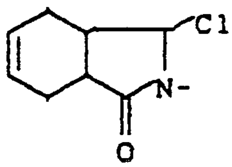 | H | H | $-CHCH_2-O-CH_2CF_3$<br>$CH_2F$ |
| 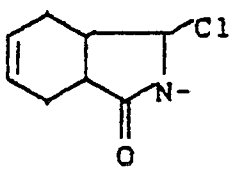 | Cl | F | $-CH_2CH_2-O-CH_2CH_2-O-CHF_2$ |
| 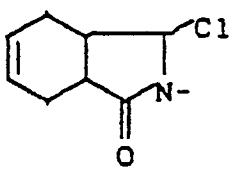 | Cl | F | $-CH_2CH_2-O-CH_2CH_2-O-CH_2CF_3$ |
| 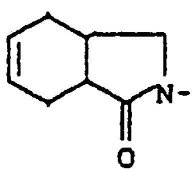 | F | F | $CH_2F$<br>$-CH_2CH_2-O-CH$<br>$CH_2F$ |
| 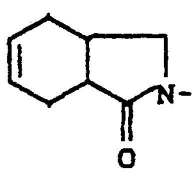 | Cl | F | $-CHCH_2-O-CH_2CF_3$<br>$CH_3$ |

Tabelle 1 - Fortsetzung

| Het | X | Y | R |
|---|---|---|---|
| 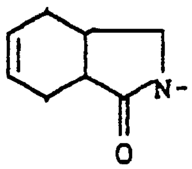 | Cl | F | $-CH_2CH_2-O-CH_2CH_2-O-CF_2CHF_2$ |
| 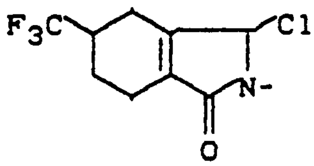 | F | F | $-CH_2CH_2-O-CH_2CH_2-O-CH_2CF_3$ |
| 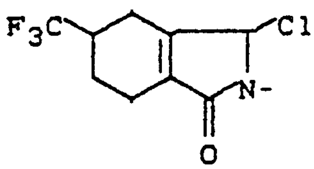 | H | H | $-CHCH_2-O-CH_2CF_3$<br>$\vert$<br>$CH_2F$ |
| 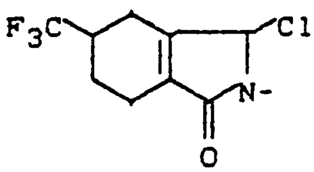 | Cl | F | $-CH_2CH_2-O-CH_2CH_2-O-CHF_2$ |
| 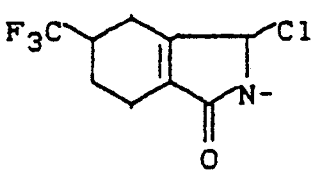 | Cl | F | $-CH_2CH_2-O-CH_2CH_2-O-CH_2CF_3$ |
| 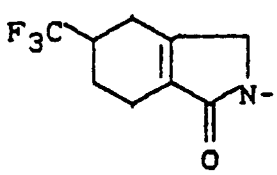 | F | F | $-CH_2CH_2-O-CH(CH_2F)(CH_2F)$ |
| 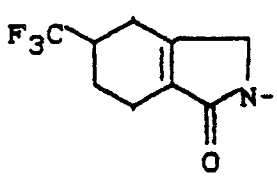 | Cl | F | $-CHCH_2-O-CH_2CF_3$<br>$\vert$<br>$CH_3$ |

Tabelle 1 - Fortsetzung

| Het | X | Y | R |
|---|---|---|---|
| 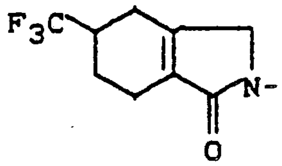 | Cl | F | $-CH_2CH_2-O-CH_2CH_2-O-CF_2CHF_2$ |
| 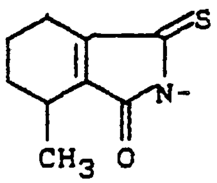 | F | F | $-CH_2CH_2-O-CH_2CH_2-O-CHF_2$ |
| 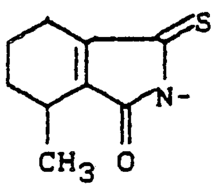 | H | H | 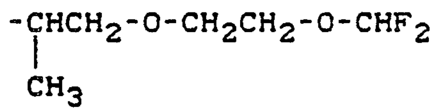 |
| 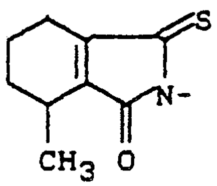 | Cl | F | $-CH_2CH_2-O-CH_2CH_2-O-CHF_2$ |
| 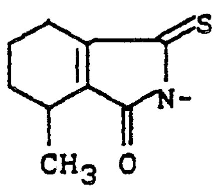 | Cl | F | $-CH_2CH_2-O-CH_2CH_2-O-CH_2CF_3$ |
| 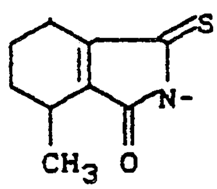 | Cl | F | 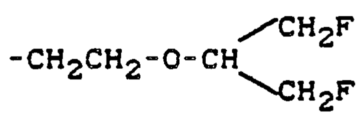 |
| 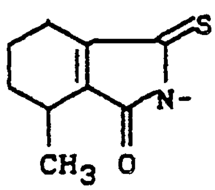 | Cl | F | $-CH_2CH_2-O-CH_2CF_3$ |

Tabelle 1 - Fortsetzung

| Het | X | Y | R |
|---|---|---|---|
| 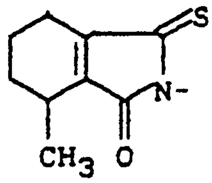 | Cl | F | $-CHCH_2-O-CH_2CF_3$<br>$\vert$<br>$CH_2F$ |
| 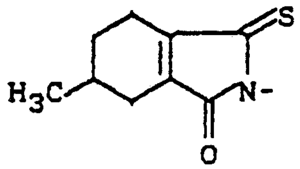 | F | F | $-CH_2CH_2-O-CH_2CH_2-O-CH_2CF_3$ |
| 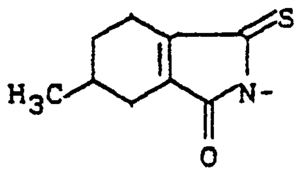 | H | H | $-CHCH_2-O-CH_2CH_2-O-CH_2CF_3$<br>$\vert$<br>$CH_3$ |
| 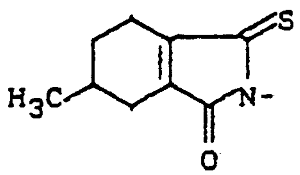 | Cl | F | $-CH_2CH_2-O-CH_2CH_2-O-CHF_2$ |
| 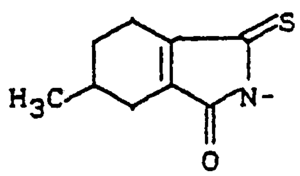 | Cl | F | $-CH_2CH_2-O-CH_2CH_2-O-CH_2CF_3$ |
| 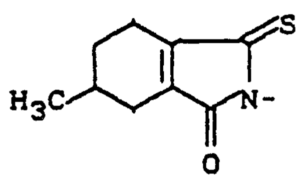 | Cl | F | $-CHCH_2-O-CHCF_3$<br>$\vert$<br>$CH_3$ |
| 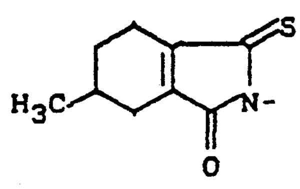 | Cl | F | $-CH_2CH_2-O-CH_2CF_3$ |

Tabelle 1 - Fortsetzung

| Het | X | Y | R |
|---|---|---|---|
| 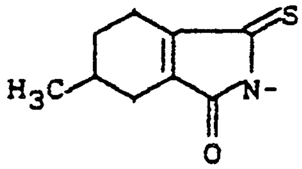 | Cl | F | $-CHCH_2-O-CHF_2$<br>$CH_3$ |
| 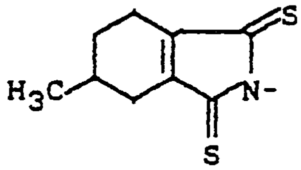 | F | F | $-CH_2CH_2-O-CH_2CH_2-O-CH_2CF_3$ |
| 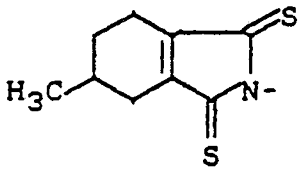 | H | H | $-CH_2CH_2-O-CH_2CH_2-O-CH_2CF_3$ |
| 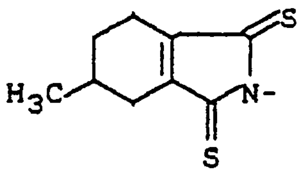 | Cl | F | $-CH_2CH_2-O-CH_2CH_2-O-CHF_2$ |
| 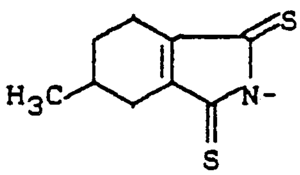 | Cl | F | $-CH_2CH_2-O-CH_2CH_2-O-CH_2CF_3$ |
| 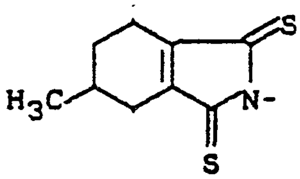 | Cl | F | $CH_2F$<br>$-CH_2CH_2-O-CH$<br>$CH_2F$ |
| 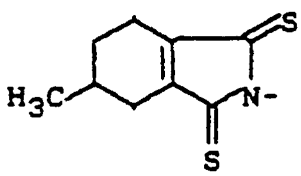 | Cl | F | $-CH_2CH_2-O-CH_2CF_3$ |

Tabelle 1 - Fortsetzung

| Het | X | Y | R |
|---|---|---|---|
| 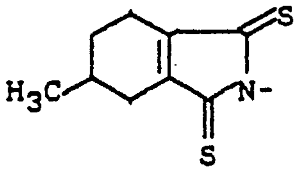 | Cl | F | $-CH(CH_3)CH_2-O-CHF_2$ |
| 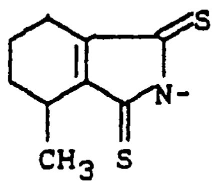 | F | F | $-CH_2CH_2-O-CH_2CH_2-O-CH_2CF_3$ |
| 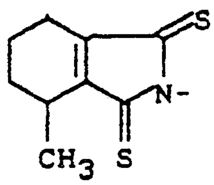 | H | H | $-CH(CH_3)CH_2-O-CH_2CH_2-O-CH_2CF_3$ |
| 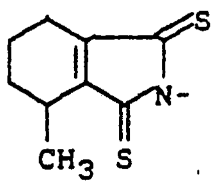 | Cl | F | $-CH_2CH_2-O-CH_2CH_2-O-CHF_2$ |
| 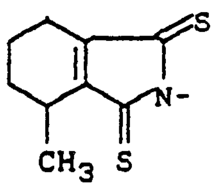 | Cl | F | $-CH(CH_2F)CH_2-O-CH_2CF_3$ |
| 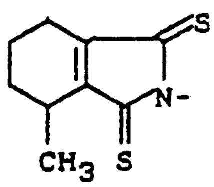 | Cl | F | $-CH_2CH_2-O-CH_2CH_2-O-CH_2CF_3$ |
| 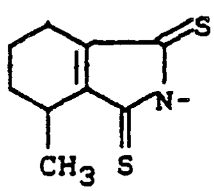 | Cl | F | $-CH_2CH_2-O-CH_2CF_3$ |

<u>Tabelle 1</u> - Fortsetzung

| Het | X | Y | R |
|---|---|---|---|
| 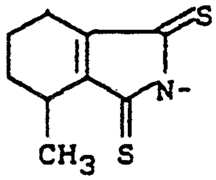 | Cl | F | 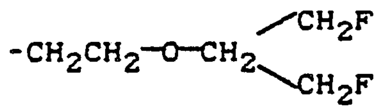 |
| 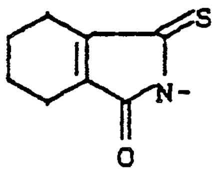 | F | F | $-CH_2CH_2-O-CH_2CH_2-O-CH_2CF_3$ |
| 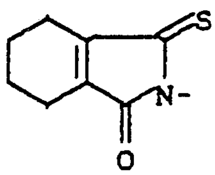 | H | H | 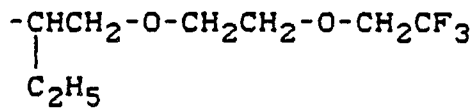 |
| 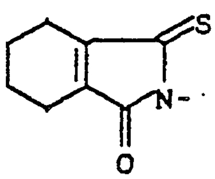 | Cl | F | $-CH_2CH_2-O-CH_2CH_2-O-CHF_2$ |
| 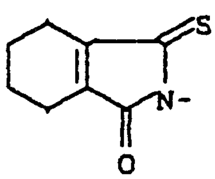 | Cl | F | $-CH_2CH_2-O-CH_2CH_2-O-CH_2CF_3$ |
| 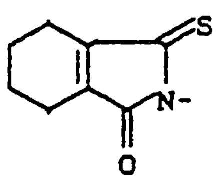 | Cl | F | 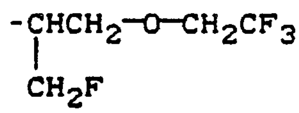 |
| 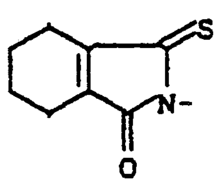 | Cl | F | $-CH_2CH_2-O-CH_2CF_3$ |

Tabelle 1 - Fortsetzung

| Het | X | Y | R |
|---|---|---|---|
| 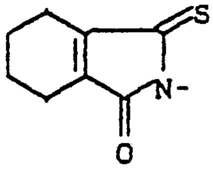 | Cl | F | $CH_2F$<br>$-CH_2CH_2-O-CH_2CH$<br>$CH_2F$ |
| 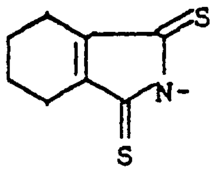 | Cl | F | $-CH_2CH_2-O-CH_2CH_2-O-CHF_2$ |
| 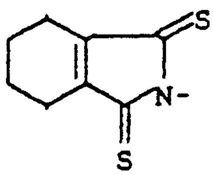 | Cl | F | $-CHCH_2-O-CH_2CH_2-O-CH_2CF_3$<br>$CH_3$ |
| 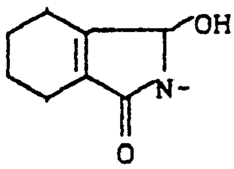 | H | H | $-CHCH_2-O-CH_2CF_3$<br>$C_2H_5$ |
| 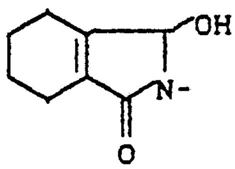 | F | F | $-CH_2CH_2-O-CH_2CH_2-O-CH_2CF_3$ |
| 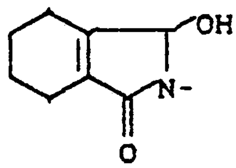 | Cl | F | $-CH_2CH_2-O-CH_2CH_2-O-CHF_2$ |
| 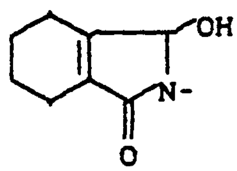 | Cl | F | $-CHCH_2-O-CH_2CF_3$<br>$CH_2F$ |

Tabelle 1 - Fortsetzung

| Het | X | Y | R |
| --- | --- | --- | --- |
| 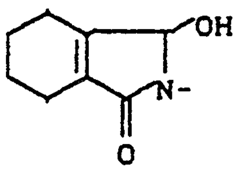 | Cl | F | $-CH_2CH_2-O-CH_2CH_2-O-CF_2CHF_2$ |
| 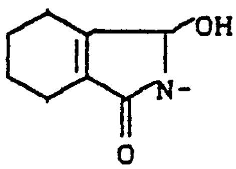 | Cl | F | $-CH_2CH_2-O-CH_2CF_3$ |
| 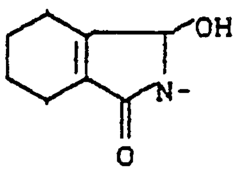 | Cl | F | $CH_2F$<br>$-CH_2CH_2-O-CH_2CH$<br>$CH_2F$ |
| 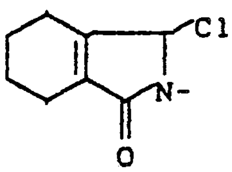 | H | H | $-CHCH_2-O-CH_2CH_2-O-CH_2CF_3$<br>$C_2H_5$ |
| 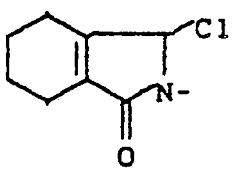 | H | H | $-CHCH_2-O-CH_2CF_3$<br>$CH_2F$ |
| 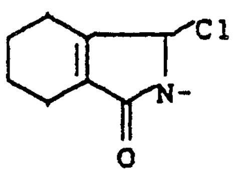 | F | F | $-CHCH_2-O-CH_2CH_2-O-CF_2CHF_2$<br>$CH_3$ |
| 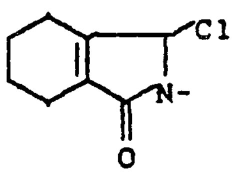 | Cl | F | $-CH_2CH_2-O-CH_2CH_2-O-CHF_2$ |

Tabelle 1 - Fortsetzung

| Het | X | Y | R |
|---|---|---|---|
| 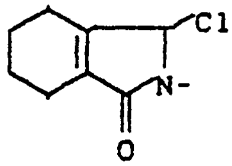 | Cl | F | $-CH_2CH_2-O-CH_2CH_2-O-CH_2CF_3$ |
| 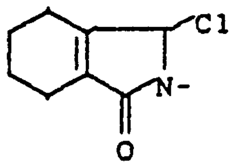 | Cl | F | $CH_2F$<br>$-CH_2CH_2-O-CH$<br>$CH_2F$ |
| 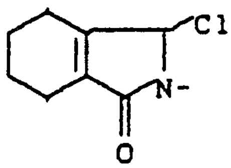 | Cl | F | $-CH_2CH_2-O-CH_2CF_3$ |
| 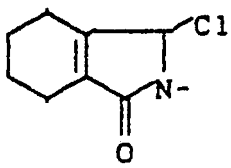 | Cl | F | $-CHCH_2-O-CHF_2$<br>$CH_3$ |
| 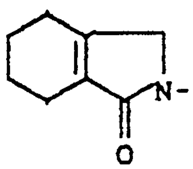 | H | H | $-CHCH_2-O-CH_2CH_2-O-CH_2CF_3$<br>$CH_3$ |
| 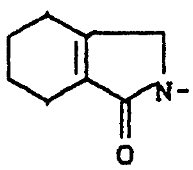 | F | F | $-CHCH_2-O-CH_2CH_2-O-CH_2CF_3$<br>$C_2H_5$ |
| 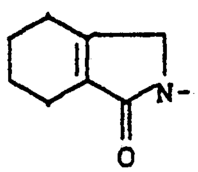 | Cl | F | $-CH_2CH_2-O-CH_2CH_2-O-CHF_2$ |

Tabelle 1 - Fortsetzung

| Het | X | Y | R |
|---|---|---|---|
| 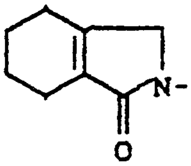 | Cl | F | $-CH_2CH_2-O-CH_2CH_2-O-CH_2CF_3$ |
| 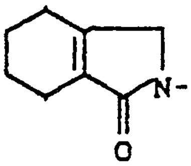 | Cl | F | $-CH_2CH_2-O-CH_2CF_3$ |
| 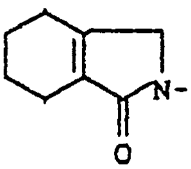 | Cl | F | $-CHCH_2-O-CHF_2$<br>$\vert$<br>$CH_3$ |
| 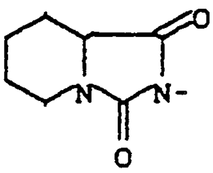 | H | H | $-CHCH_2-O-CH_2CH_2-O-CH_2CF_3$<br>$\vert$<br>$CH_3$ |
| 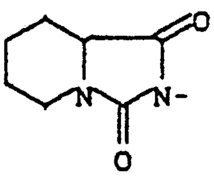 | H | H | $CH_2F$<br>$-CH_2CH_2-O-CH$<br>$CH_2F$ |
| 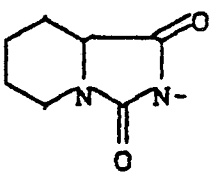 | F | F | $-CHCH_2-O-CH_2CH_2-O-CH_2CF_3$<br>$\vert$<br>$C_2H_5$ |
| 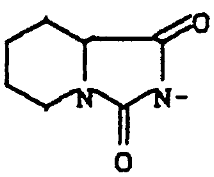 | F | F | $-CHCH_2-O-CH_2CH_2-O-CHF_2$<br>$\vert$<br>$CH_3$ |

Tabelle 1 - Fortsetzung

| Het | X | Y | R |
|---|---|---|---|
| 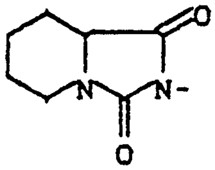 | Cl | F | $-CH_2CH_2-O-CH_2CH_2-O-CHF_2$ |
| 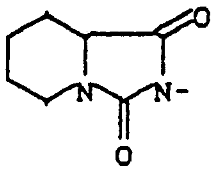 | Cl | F | $-CH_2CH_2-O-CH_2CH_2-O-CH_2CF_3$ |
| 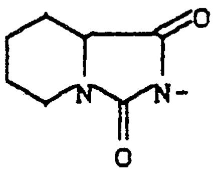 | Cl | F | $-CHCH_2-O-CH_2CF_3$<br>\|<br>$CH_2F$ |
| 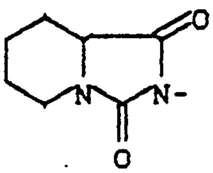 | Cl | F | $-CHCH_2-O-CH_2CH_2-O-CH_2CF_3$<br>\|<br>$CH_3$ |
| 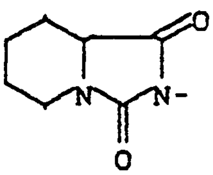 | Br | F | $-CH_2CH_2-O-CH$ ( $CH_2F$ / $CH_2F$ ) |
| 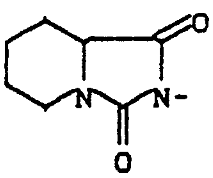 | Br | F | $-CH_2CH_2-O-CH_2CH_2-O-CHF_2$ |
| 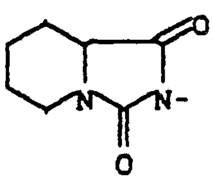 | Cl | F | $-CH_2CH_2-O-CH_2CF_3$ |

Tabelle 1 - Fortsetzung

| Het | X | Y | R |
|---|---|---|---|
| 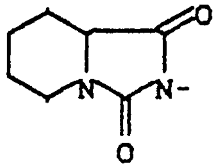 | Cl | F | $-CHCH_2-O-CHF_2$<br>$\vert$<br>$CH_3$ |
| 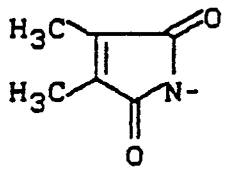 | H | H | $-CHCH_2-O-CH_2CH_2-O-CH_2CF_3$<br>$\vert$<br>$CH_2F$ |
| 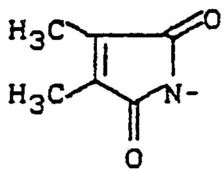 | F | F | $CH_2F$<br>$-CH_2CH_2-O-CH$<br>$CH_2F$ |
| 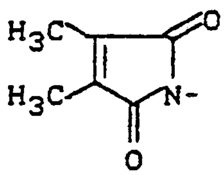 | Cl | F | $-CH_2CH_2-O-CH_2CH_2-O-CHF_2$ |
| 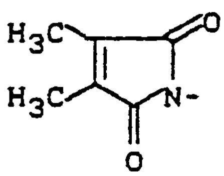 | Cl | F | $-CH_2CH_2-O-CH_2CH_2-O-CH_2CF_3$ |
| 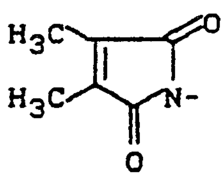 | Cl | F | $CH_2F$<br>$-CH_2CH_2-O-CH$<br>$CH_2F$ |
| 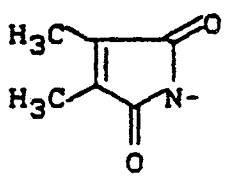 | Cl | F | $-CH_2CH_2-O-CH_2CH_2-O-CF_2CHF_2$ |

<u>Tabelle 1</u> - Fortsetzung

| Het | X | Y | R |
|---|---|---|---|
| 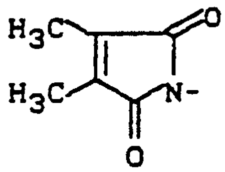 | Br | F | $-CH_2CH_2-O-CH_2CH_2-O-CHF_2$ |
| 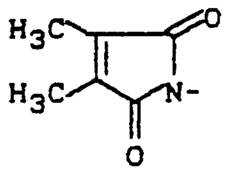 | Br | F | $-CHCH_2-O-CH_2CF_3$<br>$\vert$<br>$CH_2F$ |
| 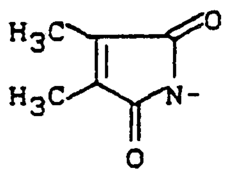 | Cl | F | $-CH_2CH_2-O-CH_2CF_3$ |
| 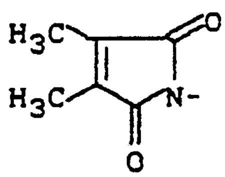 | Cl | F | $-CHCH_2-O-CHF_2$<br>$\vert$<br>$CH_3$ |
| 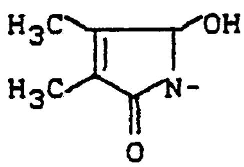 | H | H | $-CHCH_2-O-CH_2CH_2-O-CH_2CF_3$<br>$\vert$<br>$C_2H_5$ |
| 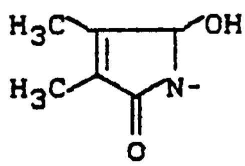 | F | F | $-CHCH_2-O-CH_2CH_2-O-CH_2CF_3$<br>$\vert$<br>$CH_3$ |
| 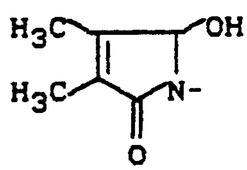 | Cl | F | $-CH_2CH_2-O-CH_2CH_2-O-CHF_2$ |

Tabelle 1 - Fortsetzung

| Het | X | Y | R |
|---|---|---|---|
| 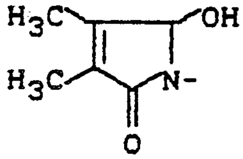 | Cl | F | $-CH_2CH_2-O-CH_2CH_2-O-CH_2CF_3$ |
| 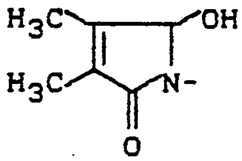 | Cl | F | $-CHCH_2-O-CH_2CF_3$<br>$CH_2F$ |
| 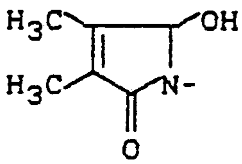 | Cl | F | $-CHCH_2-O-CH_2CF_3$<br>$CH_3$ |
| 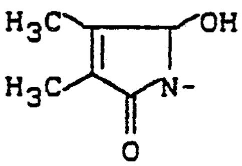 | Cl | F | $-CH_2CH_2-O-CH_2CF_3$ |
| 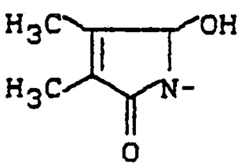 | Cl | F | $-CHCH_2-O-CHF_2$<br>$CH_3$ |
| 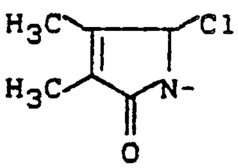 | H | H | $-CHCH_2-O-CH_2CH_2-O-CHF_2$<br>$C_2H_5$ |
| 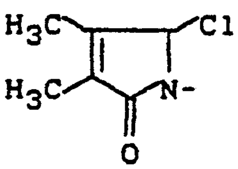 | F | F | $-CHCH_2-O-CH_2CF_3$<br>$CH_3$ |

Tabelle 1 - Fortsetzung

| Het | X | Y | R |
|---|---|---|---|
| 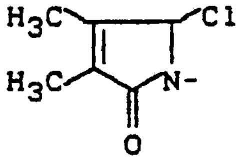 | Cl | F | $-CH_2CH_2-O-CH_2CH_2-O-CHF_2$ |
| 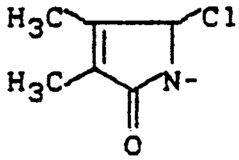 | Cl | F | $-CH_2CH_2-O-CH_2CH_2-O-CH_2CF_3$ |
| 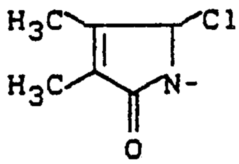 | Cl | F | 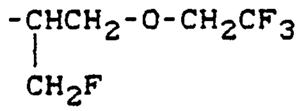 |
| 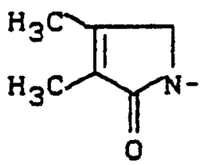 | H | H | 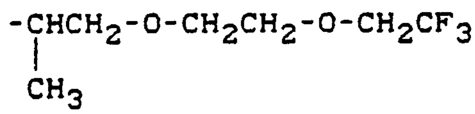 |
| 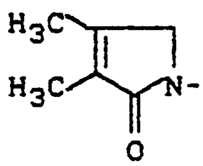 | F | F | $-CH_2CH_2-O-CH_2CH_2-O-CH_2CF_3$ |
| 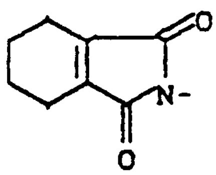 | Cl | F | 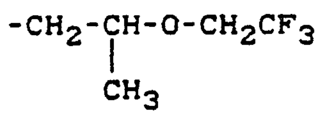 |
| 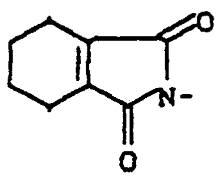 | Br | F | 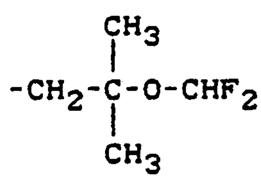 |

Tabelle 1 - Fortsetzung

| Het | X | Y | R |
|---|---|---|---|
| 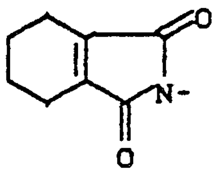 | H | H | 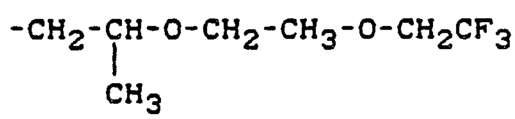 |
| 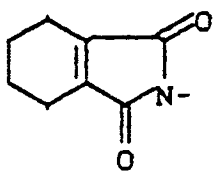 | Br | F | 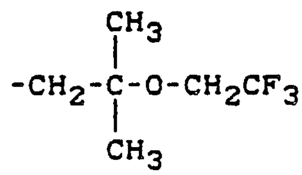 |
| 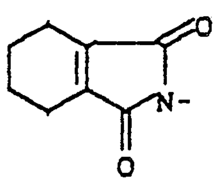 | H | H | 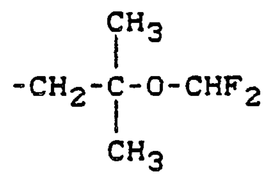 |

Verwendet man beispielsweise 3,4,5,6-Tetrahydrophthalsäureanhydrid und 2,4-Difluor-5-(2-(2-difluormethoxyethoxy)-ethoxy)-anilin als Ausgangsstoffe, so kann der Reaktionsablauf beim erfindungsgemäßen Verfahren (a) durch das folgende Formelschema wiedergegeben werden:

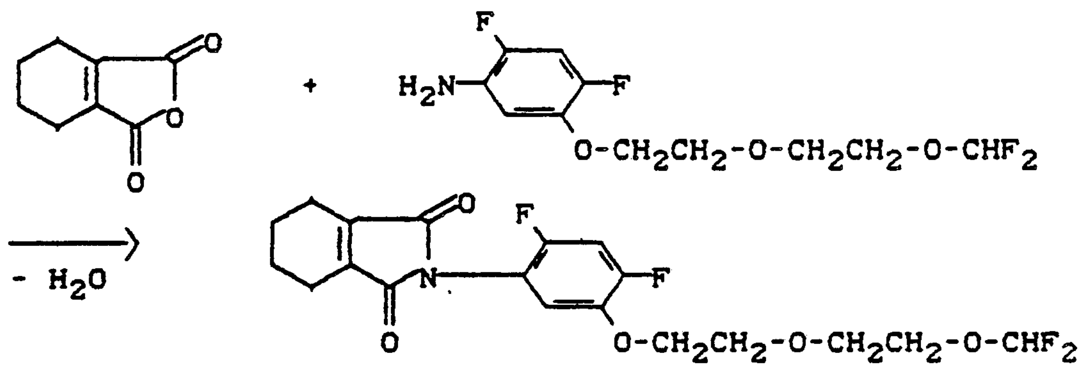


Verwendet man beispielsweise N-(4-Brom-2-fluor-5-(2-(2-(2,2,2-trifluorethoxy)-ethoxy)-ethoxy)-phenyl)-dimethylmaleinsäureimid und Natriumboranat als Ausgangsstoffe, so kann der Reaktionsablauf beim erfindungsgemäßen Verfahren (b) durch das folgende Formelschema wiedergegeben werden:

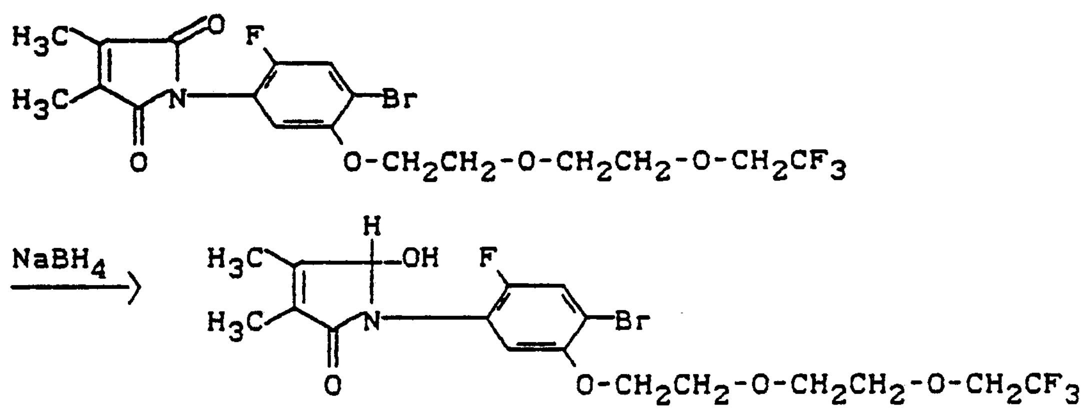


Verwendet man beispielsweise N-(4-Chlor-2-fluor-5-(2-(2,2,2-trifluorethoxy)-ethoxy)-phenyl)-3,4,5,6-tetrahydrophthalimid und Phosphor(V)-sulfid als Ausgangsstoffe, so kann der Reaktionsablauf beim erfindungsgemäßen Verfahren (c) durch das folgende Formelschema wiedergegeben werden:

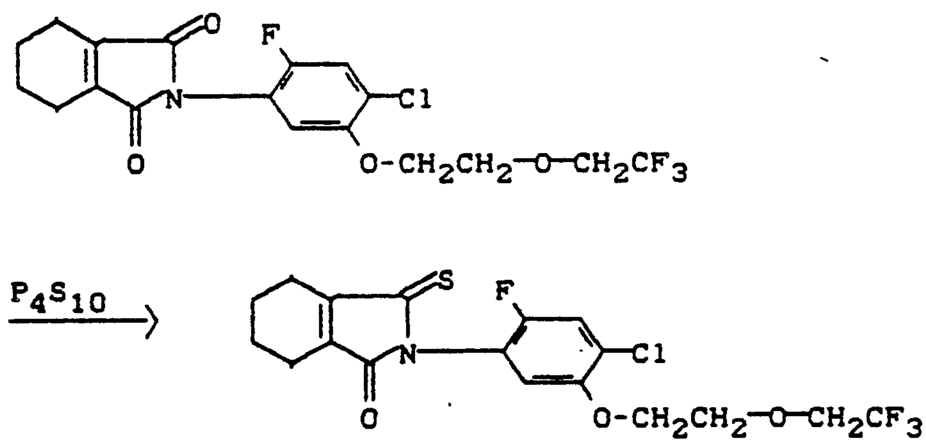


Verwendet man beispielsweise N-(4-Chlor-2-fluor-(5-(2-difluormethoxy-ethoxy)-phenyl)-3,4-dimethyl-$\Delta^3$-pyrrolin-5-ol-2-on und Thionylchlorid als Ausgangsstoffe, so läßt sich der Reaktionsablauf beim erfindungsgemäßen Verfahren (d) durch das folgende Formelschema wiedergeben:

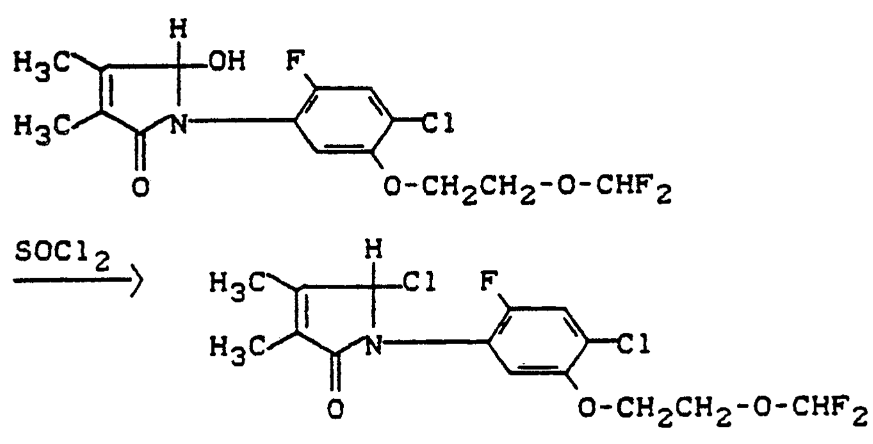


Verwendet man beispielsweise N-(2,4-Difluor-5-(2-(1,1,2,2-tetrafluorethoxy)-ethoxy)-phenyl)-5-chlor-3,4-dimethyl-$\Delta^3$-pyrrolin-2-on als Ausgangsverbindung, so kann der Reaktionsablauf beim erfindungsgemäßen Verfahren (e) durch das folgende Formelschema wiedergegeben werden:

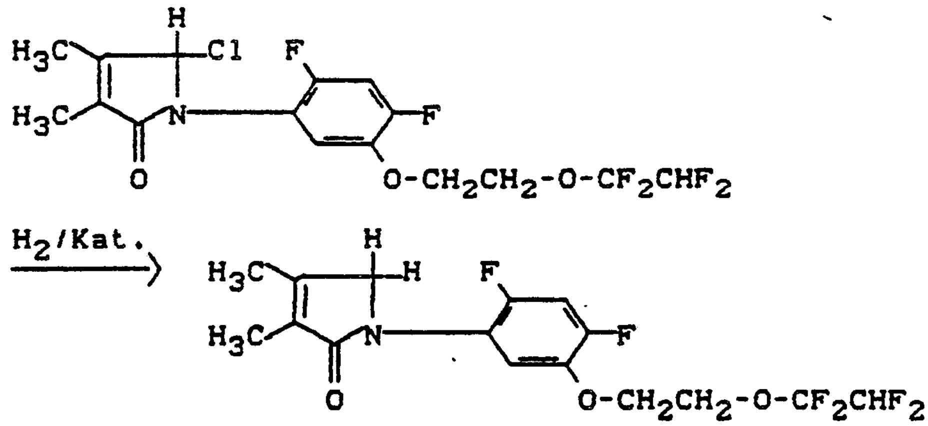


Verwendet man beispielsweise N-(2-Fluor-5-hydroxy-phenyl)-3,4,5,6-tetrahydro-phthalimid und Methansulfonsäure-(2-(2-difluormethoxy-ethoxy)-ethyl)-ester als Ausgangsstoffe, so kann der Reaktionsablauf beim erfindungsgemäßen Verfahren (f) durch das folgende Formelschema wiedergegeben werden:

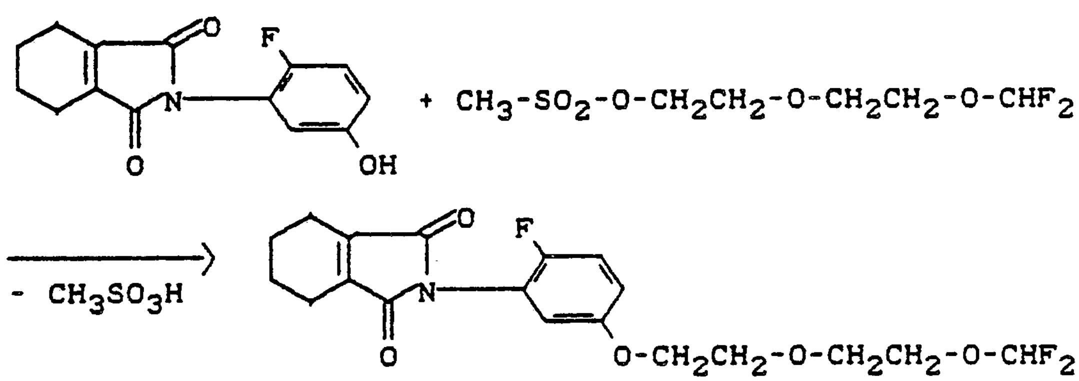


Verwendet man beispielsweise N-(2-Fluor-5-(2-(2-difluormethoxyethoxy)-ethoxy)-phenyl)-phthalimid und Chlor als Ausgangsstoffe, so kann der Reaktionsablauf beim erfindungsgemäßen Verfahren (g) durch das folgende Formelschema wiedergegeben werden:

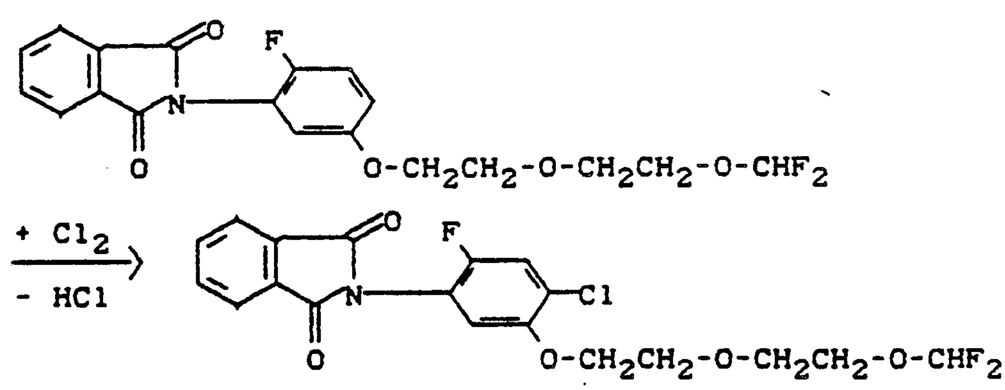


Verwendet man beispielsweise Chlorameisensäuremethylester, 4-Chlor-2-fluor-5-(2-(2-difluormethoxy-ethoxy)- ethoxy)-anilin und Piperidin-2-carbonsäure-ethylester als Ausgangsstoffe, so kann der Reaktionsablauf beim erfindungsgemäßen Verfahren (h) durch das folgende Formelschema wiedergegeben werden:

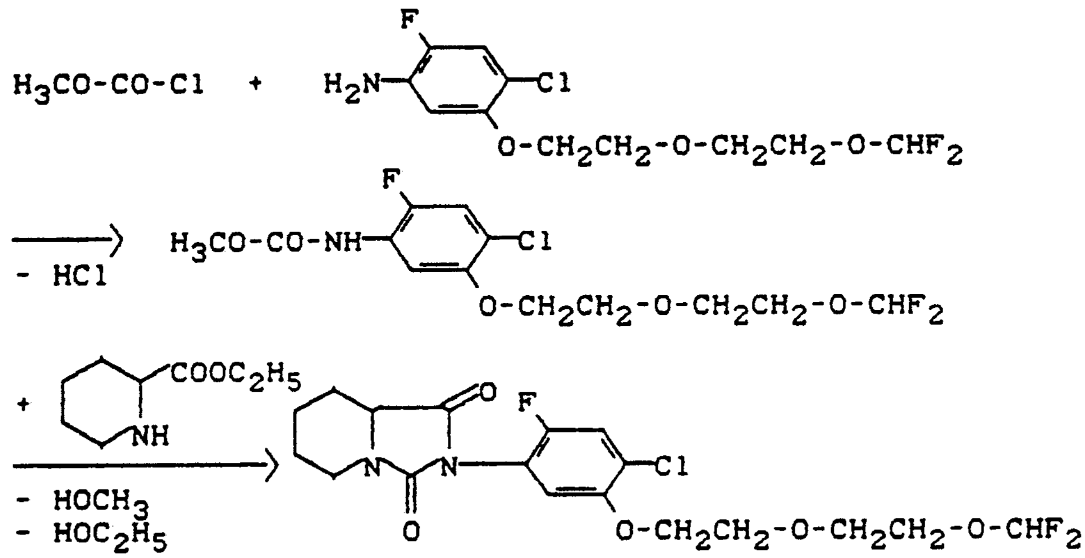


Die beim erfindungsgemäßen Verfahren (a) zur Herstellung von Verbindungen der Formel (I) als Ausgangsstoffe zu verwendenden cyclischen Anhydride sind durch die Formel (II) allgemein definiert.

In Formel (II) hat A vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für A angegeben wurde.

Als Beispiele für die Ausgangsstoffe der Formel (II) seien genannt:

Phthalsäureanhydrid, 3,4,5,6-Tetrahydro-phthalsäureanhydrid, 3-Methyl-, 4-Methyl-, 4-Trifluormethyl- und 3,3- Dimethyl-3,4,5,6-tetrahydro-phthalsäureanhydrid, 1,2,3,4-Tetrahydro-, 1,2,3,6-Tetrahydro- und 2,3,4,5-Tetrahydro-phthalsäureanhydrid, 3,6-Dihydro-phthalsäureanhydrid und Dimethylmaleinsäureanhydrid.

Die Ausgangsstoffe der Formel (II) sind bekannte organische Synthesechemikalien.

Die beim erfindungsgemäßen Verfahren (a) zur Herstellung von Verbindungen der Formel (I) weiter als Ausgangsstoffe zu verwendenden Arylamine sind durch die Formel (III) allgemein definiert.

In Formel (III) haben R, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R, X und Y angegeben wurden.

Als Beispiele für die Ausgangsstoffe der Formel (III) seien genannt:

5-(2-(2,2,2-Trifluorethoxy)-ethoxy)-, 5-(2-(2-(2,2,2-Trifluorethoxy)-ethoxy)-ethoxy)-, 5-(2-(2,2-Bis-fluormethyl-ethoxy)-ethoxy)-, 5-(1-Methyl-2-(2,2,2-trifluorethoxy)-ethoxy)-, 5-(1-Ethyl-2-(2,2,2-trifluorethoxy)-ethoxy)-, 5-(1-Fluormethyl-2-(2,2,2-trifluorethyl)-ethoxy)-, 5-(2-Difluormethoxy-ethoxy)- und 5-(2-(2-Difluormethoxy-ethoxy)-ethoxy)-, -2-fluor-anilin, -4-fluor-anilin, -2,4-difluor-anilin, -2-fluor-4-chlor-anilin und -2-fluor-4-bromanilin.

Die Ausgangsstoffe der Formel (III) sind noch nicht aus der Literatur bekannt.

Man erhält die neuen Verbindungen der allgemeinen Formel (III), wenn man

(α) Hydroxyarylamine der allgemeinen Formel (IX)

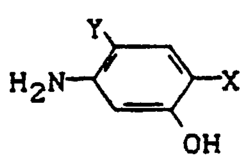

(IX)

in welcher

X und Y die oben angegebenen Bedeutungen haben,

mit Alkylierungsmitteln der allgemeinen Formel (V)

$X^1$ - R (V)

in welcher

R und $X^1$ die oben angegebenen Bedeutungen haben,

in Gegenwart eines Verdünnungsmittels, wie z. B. Aceton, Acetonitril, Dimethylformamid, Dimethylsulfoxid oder N-Methyl-pyrrolidon, gegebenenfalls in Gegenwart eines Säureakzeptors, wie z. B. Natrium- oder Kalium-carbonat, -hydrid oder -hydroxid, sowie gegebenenfalls zusätzlich in Gegenwart von Wasser, bei Temperaturenzwischen 0 °C und 100 °C umsetzt, oder wenn man

(β) Nitrophenol-Derivate der allgemeinen Formel (X)

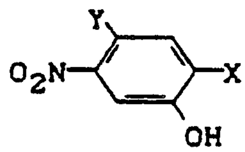

(X)

in welcher
X und Y die oben angegebenen Bedeutungen haben,
mit Alkylierungsmitteln der allgemeinen Formel (V)

$X^1$ - R (V)

in welcher
R und $X^1$ die oben angegebenen Bedeutungen haben,
nach der oben unter (α) angegebenen Methodik umsetzt und die so erhaltenen Verbindungen der allgemeinen Formel (XI)

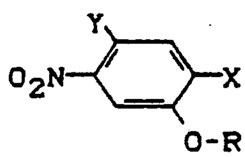

(XI)

in welcher
R, X und Y die oben angegebenen Bedeutungen haben,
nach üblichen Methoden, beispielsweise mit Wasserstoff in Gegenwart eines Katalysators, wie z. B. Platin auf Aktivkohle, in Gegenwart eines Verdünnungsmittels, wie z. B. Ethanol, bei Temperaturen zwischen 0 °C und 100 °C reduziert, oder wenn man

(γ) Phenol-Derivate der allgemeinen Formel (XII)

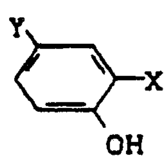

(XII)

in welcher
X und Y die oben angegebenen Bedeutungen haben,
mit Alkylierungsmitteln der allgemeinen Formel (V)

$X^1$ - R (V)

in welcher
R und $X^1$ die oben angegebenen Bedeutungen haben,
nach der oben unter (α) angegebenen Methode umsetzt und die so erhaltenen Verbindungen der Formel (XIII)

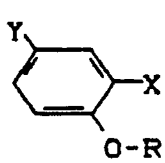

(XIII)

in welcher
R, X und Y die oben angegebenen Bedeutungen haben,
mit Salpetersäure zu den Verbindungen der For mel (XI) nitriert und anschließend nach üblichen Methoden (siehe (β)) reduziert.

Die Nitrierung kann durchgeführt werden in anorganischen Säuren, wie Schwefelsäure oder Salpetersäure, aber auch in organischen Lösungsmitteln, vorzugsweise Halogenkohlenwasserstoffen, wie Methylenchlorid, mit oder ohne Zusatz von Salzen der salpetrigen Säure, mit oder ohne Zusatz von Harnstoff oder Amidosulfonsäure, bei Temperaturen von -30 °C bis +60 °C, vorzugsweise -10 °C bis +30 °C.

Die als Ausgangsstoffe benötigten Phenole der Formel (XII), die Hydroxyarylamine der Formel (IX) und die Nitrophenol-Derivate der Formel (X) sind bereits bekannt (vgl. EP-A 61 741).
Als Beispiele hierfür seien genannt:
2-Chlor-4-fluorphenol und 4-Fluorphenol, 2-Fluor-3-hydroxy-anilin und -nitrobenzol, 4-Chlor-2-fluor-3-hydroxy-anilin und -nitrobenzol sowie 4-Brom-2-fluor-3-hydroxy-anilin und -nitrobenzol.

Die weiter als Ausgangsstoffe benötigten Alkylierungsmittel sind durch die Formel (V) allgemein definiert.
In Formel (V) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde und $X^1$ steht vorzugsweise für Chlor, Brom, Methylsulfonyloxy, Phenylsulfonyloxy oder Tolylsulfonyloxy.

Als Beispiele für die Ausgangsstoffe der Formel (V) seien genannt:
2-(2,2,2-Trifluorethoxy)-ethyl-, 2-(2-(2,2,2-Trifluorethoxy)-ethoxy)-ethyl-, 2-(2,2-Bis-fluormethyl-ethoxy)-ethyl-, 2-(2,2,2-Trifluorethoxy)-1-methyl-ethyl-, 2-(2,2,2-Trifluorethoxy)-1-ethyl-ethyl-, 1-Fluormethyl-2-(2,2,2-trifluorethoxy)-ethyl-, 2-Difluormethoxy-ethyl-und 2-(2-Difluormethoxy-ethoxy)-ethyl- -chlorid und -bromid; ferner Methansulfonsäure-, Benzolsulfonsäure-und p-Toluolsulfonsäure- -2-(2,2,2-trifluorethoxy)-ethylester, -2-(2-(2,2,2-trifluorethoxy)-ethoxy)-ethylester, -2-(2,2-bis-fluormethyl-ethoxy)-ethylester, -2-(2,2,2-trifluorethoxy)-1-methyl-ethylester, -2-(2,2,2-trifluorethoxy)-1-ethyl-ethylester, -1-fluormethyl-2-(2,2,2-trifluorethoxy)-ethylester, -2-difluormethoxy-ethylester und -2-(2-difluormethoxy-ethoxy)-ethylester.

Die Ausgangsstoffe der Formel (V) sind noch nicht aus der Literatur bekannt.

Man erhält die neuen Verbindungen der Formel (V) beispielsweise, wenn man entsprechende Alkohole der Formel (XIV)
HO—R (XIV)
in welcher
R die oben angegebene Bedeutung hat,

α) für den Fall, daß $X^1$ für Chlor oder Brom steht, mit einen Chlorierungsmittel, wie z. B. Thionylchlorid, Phosphor(III)-chlorid, Phosphor(V)-chlorid und/oder Phosphorylchlorid, oder mit einem Bromierungsmittel, wie z. B. Phosphor(III)-bromid, gegebenenfalls in Gegenwart einer basischen Verbindung, wie z. B. Pyridin, und gegebenenfalls in Gegenwart eines Verdünnungsmittels, wie z. B. Diethylether, bei Temperaturen zwischen -10 °C und +120 °C umsetzt, oder

β) für den Fall, daß $X^1$ für Methylsulfonyloxy, Phenylsulfonyloxy oder Tolylsulfonyloxy steht, mit Methansulfonsäurechlorid, Benzolsulfonsäurechlorid oder Toluolsulfonsäurechlorid, gegebenenfalls in Gegenwart einer basischen Verbindung, wie z. B. Pyridin, und gegebenenfalls in Gegenwart eines Verdünnungsmittels, wie z. B. Methylenchlorid, bei Temperaturen zwischen -20 °C und +60 °C umsetzt.

Die als Zwischenprodukte benötigten Alkohole sind durch die Formel (XIV) allgemein definiert. In Formel (XIV) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde.

Als Beispiele für die Zwischenprodukte der Formel (XIV) seien genannt:
2-(2,2,2-Trifluorethoxy)-ethanol, 2-(2-(2,2,2-Trifluorethoxy)-ethoxy)-ethanol, 2-(2,2-Bis-fluormethyl-ethoxy)-ethanol, 2-(2,2,2-Trifluorethoxy)-1-methyl-ethanol, 2-(2,2,2-Trifluorethoxy)-1-ethyl-ethanol, 1-Fluormethyl-2-(2,2,2-trifluorethoxy)-ethanol, 2-(2-Difluormethoxy-ethoxy)-ethanol und 2-Difluormethoxy-ethanol.

Die Alkohole der Formel (XIV) sind teilweise bekannt (vgl. J. Am. Chem Soc. 79 (1957); US-P 3394115).

Neu sind die Alkohole der Formel (XIV), bei welchen R für verzweigtes und wenigstens durch 1 Sauerstoffatom unterbrochenes und wenigstens durch 1 Fluoratom substituiertes Alkyl steht.

Bevorzugt sind die neuen Alkohole der Formel (XIV), bei denen R für verzweigtes und durch 2 bis 4 Fluoratome substituiertes Oxaalkyl oder Dioxaalkyl mit bis zu 10 Kohlenstoffatomen steht. Die neuen Alkohole werden im folgenden mit der Formel (XIVa) bezeichnet.

Als Beispiele für die neuen Verbindungen der Formel (XIV) seien genannt:
1-Methyl-2-(2,2,2-trifluorethoxy)-ethanol, 1-Ethyl-2-(2,2,2-trifluorethoxy)-ethanol, 1-Fluormethyl-2-(2,2,2-tri-

fluorethoxy)-ethanol und 2-(2-Fluor-1-fluormethyl-ethoxy)-ethanol.

Man erhält die neuen Verbindungen der Formel (XIVa), wenn man geeignete Alkohole, wie z. B. 2-Fluor-1-fluormethyl- ethanol(1,3-Difluor-2-propanol) oder 2,2,2-Trifluorethanol, mit geeigneten Oxiranen, wie z. B. Ethylenoxid, Propylenoxid, Butylenoxid oder Epifluorhydrin, in Gegenwart von Basen, wie z. B. Natrium-oder Kaliumhydroxid, bei Temperaturen zwischen -80 °C und +12 °C und bei Drukken zwischen 1 000 hPa und 10 000 hPa umsetzt (vgl. Herstellungsbeispiele).

Die bei den erfindungsgemäßen Verfahren (b) und (c) zur Herstellung von Verbindungen der Formel (I) als Ausgangsstoffe zu verwendenden substituierten Arylimide sind durch die Formel (Ia) allgemein definiert.

In Formel (Ia) haben A, R, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, X und Y angegeben wurden.

Beispiele für die Ausgangsstoffe der Formel (Ia) sind Tabelle 1 (oben) zu entnehmen.

Die substituierten Arylimide der Formel (Ia) sind neue, erfindungsgemäße Verbindungen; sie können nach dem erfindungsgemäßen Verfahren (a) hergestellt werden.

Die beim erfindungsgemäßen Verfahren (d) zur Herstellung von Verbindungen der Formel (I) als Ausgangsstoffe zu verwendenden N-Aryl-Stickstoffheterocyclen sind durch die Formel (Ib) allgemein definiert.

In Formel (Ib) haben A, R, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, X und Y angegeben wurden.

Beispiele für die Ausgangsstoffe der Formel (Ib) sind Tabelle 1 (oben) zu entnehmen.

Die N-Aryl-Stickstoffheterocyclen der Formel (Ib) sind neue, erfindungsgemäße Verbindungen; sie können nach dem erfindungsgemäßen Verfahren (b) hergestellt werden.

Die beim erfindungsgemäßen Verfahren (e) zur Herstellung von Verbindungen der Formel (I) als Ausgangsstoffe zu verwendenden N-Aryl-Stickstoffheterocyclen sind durch die Formel (Ic) allgemein definiert.

In Formel (Ic) haben A, R, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R, X und Y angegeben wurden.

Beispiele für die Ausgangsstoffe der Formel (Ic) sind Tabelle 1 (oben) zu entnehmen.

Die N-Aryl-Stickstoffheterocyclen der Formel (Ic) sind neue, erfindungsgemäße Verbindungen; sie können nach dem erfindungsgemäßen Verfahren (d) hergestellt werden.

Die beim erfindungsgemäßen Verfahren (f) zur Herstellung von Verbindungen der Formel (I) als Ausgangsstoffe zu verwendenden Hydroxyarylimide sind durch die Formel (IV) allgemein definiert.

In Formel (IV) haben A, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, X und Y angegeben wurden.

Als Beispiele für die Ausgangsstoffe der Formel (IV) seien genannt:

N-(2-Fluor-5-hydroxy-phenyl)-, N-(2,4-Difluor-5-hydroxy-phenyl)-, N-(4-chlor-2-fluor-5-hydroxy-phenyl)-phthalsäureimid, N-(2-Fluor-5-hydroxy-phenyl)-, N-(2,4-Difluor-5-hydroxy-phenyl)-, N-(4-Chlor-2-fluor-5-hydroxyphenyl)- und N-(4-Brom-2-fluor-5-hydroxy-phenyl)-3,4,5,6-tetrahydrophthalimid, N-(2-Fluor-5-hydroxy-phenyl)-, N-(2,4-Difluor-5-hydroxy-phenyl)-, N-(4-Chlor-2-fluor-5-hydroxy-phenyl)- und N-(2-Fluor-4-brom-5-hydroxy-phenyl)-, -3-methyl-, -4-methyl-, -4-trifluormethyl- und -3,3-dimethyl-3,4,5,6-tetrahydrophthalimid, N-(2-Fluor-5-hydroxy-phenyl)-, N-(2,4-Difluor-5-hydroxy-phenyl)-, N-(4-Chlor-2-fluor-3-hydroxy-phenyl)- und N-(4-Brom-2-fluor-5-hydroxy-phenyl )-3,6-dihydro-phthalimid, sowie N-(2-Fluor-5-hydroxy-phenyl)-, N-(2,4-Difluor-5-hydroxy-phenyl-, N-(4-Chlor-2-fluor-5-hydroxy-phenyl)- und N-(4-Brom-2-fluor-5-hydroxy-phenyl)-dimethylmaleinsäureimid.

Die Ausgangsstoffe der Formel (IV) sind bekannt und/oder können nach an sich bekannten Verfahren hergestellt werden (vgl. EP-A 61 741).

Die beim erfindungsgemäßen Verfahren (f) zur Herstellung von Verbindungen der Formel (I) weiter als Ausgangsstoffe zu verwendenden Alkylierungsmittel sind durch die Formel (V) allgemein definiert.

In Formel (V) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde und $X^1$ steht vorzugsweise für Chlor, Brom, Iod, Methylsulfonyl, Phenylsulfonyl oder Tolylsulfonyl.

Beispiele für die Ausgangsstoffe der Formel (V) wurden bereits oben im Zusammenhang mit der Beschreibung der Ausgangsstoffe für das erfindungsgemäße Verfahren (a) genannt.

Die beim erfindungsgemäßen Verfahren (g) zur Herstellung von Verbindungen der Formel (I) als

Ausgangsstoffe zu verwendenden substituierten Arylimide sind durch die Formel (Id) allgemein definiert.

In Formel (Id) haben A, R und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R und Y angegeben wurden.

Beispiele für die Ausgangsstoffe der Formel (Id) sind Tabelle 1 (oben) zu entnehmen.

Die substituierten Arylimide der Formel (Id) sind neue, erfindungsgemäße Verbindungen, sie können nach dem erfindungsgemäßen Verfahren (a) hergestellt werden.

Die beim erfindungsgemäßen Verfahren (h) zur Herstellung von Verbindungen der Formel (I) als Ausgangsstoffe zu verwendenden Arylamine sind durch die Formel (III) allgemein definiert.

In Formel (III) haben R, X und Y vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R, X und Y angegeben wurden.

Beispiele für die Ausgangsstoffe der Formel (III) und Verfahren zu ihrer Herstellung wurden bereits oben im Zusammenhang mit der Beschreibung der Ausgangsstoffe für das erfindungsgemäße Verfahren (a) angegeben.

Die beim erfindungsgemäßen Verfahren (h) weiter als Ausgangsstoffe zu verwendenden Chlorameisensäureester sind durch die Formel (VI) allgemein definiert. In Formel (VI) steht $R^3$ vorzugsweise für Methyl, Benzyl oder Phenyl.

Als Beispiele für die Ausgangsstoffe der Formel (VI) seien genannt:
Chlorameisensäure-methylester, -benzylester und -phenylester.

Die Chlorameisensäureester der Formel (VI) sind bekannte organische Synthesechemikalien.

Die beim erfindungsgemäßen Verfahren (h) ferner als Ausgangsstoffe zu verwendenden Piperidin-2-carbonsäureester sind durch die Formel (VIII) allgemein definiert.

In Formel (VIII) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurde und $R^4$ steht vorzugsweise für Methyl oder Ethyl.

Als Beispiele für die Ausgangsstoffe der Formel (VIII) seien genannt:
Piperidin-2-carbonsäure-methylester und -ethylester.

Die Ausgangsstoffe der Formel (VIII) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Ben zol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Carbonsäuren wie Ameisensäure, Essigsäure oder Propionsäure, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (a) kann gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N′-Dicyclohexylcarbodiimid oder übliche Acylierungskatalysatoren, wie beispielsweise 4-(N,N-Dimethylamino)-pyridin als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (a) setzt man pro Mol an Anhydrid der Formel (II) im allgemeinen 1.0 bis 1.5 Mol, vorzugsweise 1.0 bis 1.2 Mol an Amin der Formel (III) und gegebenenfalls 0.01 bis 1.2 Mol, vorzugsweise 0.1 bis 1.0 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Als Reduktionsmittel zur Durchführung des erfindungsgemäßen Verfahrens (b) kommen alle üblichen für derartige Reduktionsreaktionen verwendbaren Reduktionsmittel infrage. Vorzugsweise verwendet man komplexe Hydride, wie beispielsweise Natriumborhydrid, Natriumcyanoborhydrid, Lithiumborhydrid oder

Lithiumaluminiumhydrid.

Als Verdünnungsmittel zur Durchführung des erfindungsgemäßen Verfahrens (b) kommen in Abhängigkeit vom verwendeten Reduktionsmittel alle üblichen organischen oder anorganischen Lösungsmittel in Frage. Vorzugsweise ver wendet man Ether, wie Diethylether, Dioxan oder Tetrahydrofuran oder Alkohole, wie Methanol, Ethanol oder Propanol.

Die Reaktionstemperaturen können bei der Durchführung des erfindungsgemäßen Verfahrens (b) in Abhängigkeit vom verwendeten Reduktionsmittel in einem größeren Bereich variiert werden. Im allgemeinen arbeitet man bei Temperaturen zwischen - 20 °C und + 150 °C, vorzugsweise bei Temperaturen zwischen 0 °C und 120 °C.

Zur Durchführung des erfindungsgemäßen Verfahrens (b) setzt man pro Mol an substituiertem Arylimid der Formel (Ia) im allgemeinen 0.1 bis 2.0 Mol, vorzugsweise 0.25 bis 1.5 Mol an Reduktionsmittel ein.
Die Reaktionsdurchführung, Aufarbeitung und Isolierung der Reaktionsprodukte erfolgt nach allgemein üblichen Methoden.

Als Schwefelungsmittel zur Durchführung des erfindungsgemäßen Verfahrens (c) kommen alle üblichen für derartige Schwefelungsreaktionen verwendbaren Schwefelungsmittel in Betracht. Vorzugsweise verwendet man Phosphor-Schwefel-Verbindungen, wie beispielsweise Phosphor(V)-sulfid ($P_4S_{10}$) oder das sogenannte Lawesson-Reagenz (2,4-Bis-(4-methoxy-phenyl)-1,3-dithia-phosphetan-2,4-disulfid.

Als Verdünnungsmittel zur Durchführung des erfindungsgemäßen Verfahrens (c) kommen inerte organische Lösungsmittel infrage. Hierzu gehoren insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 180 °C, vorzugsweise bei Temperaturen zwischen 20 °C und 150 °C.

Zur Durchführung des erfindungsgemäßen Verfahrens (c) setzt man je Mol Arylimid der Formel (Ia) im allgemeinen zwischen 0,2 und 2,0 Mol, vorzugsweise zwischen 0,5 und 1,5 Mol, Schwefelungsmittel ein.

Die Reaktionsdurchführung, Aufarbeitung und Isolierung der Reaktionsprodukte erfolgt nach an sich bekannter Verfahrensweise (vgl. Bull. Soc. Chim. Belg. 87 (1978), 223 - 228). Im allgemeinen erhält man hierbei Gemische aus einfach und zweifach geschwefelten Produkten, die sich nach üblichen Trennmethoden (z. B. Chromatographie oder Kristallisation) auftrennen lassen.

Als Verdünnungsmittel zur Durchführung des erfindungsgemäßen Verfahrens (d) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Nitrile wie Acetonitril oder Propionitril.

Das erfindungsgemäße Verfahren (d) kann gegebenenfalls in Gegenwart eines geeigneten Reaktionshilfsmittels durchgeführt werden. Als solche kommen insbesondere organische Amine oder Amide infrage. Vorzugsweise verwendet man Pyridin, Dimethylanilin oder Dimethylformamid.

Die Reaktionstemperaturen können bei der Durchführung des erfindungsgemäsen Verfahrens (4) in einem größeren Bereich variiert werden. Im allgemeinen arbeitet man bei Temperaturen zwischen 0 °C und 120 °C, vorzugsweise bei Temperaturen zwischen 20 °C und 80 °C.

Zur Durchführung des erfindungsgemäßen Verfahrens (d) setzt man pro Mol an N-Aryl-Stickstoffheterocyclus der Formel (Ib) im allgemeinen 1.0 bis 5.0 Mol, vorzugsweise 1.0 bis 2.0 Mol an Thionylchlorid und gegebenenfalls 0.1 bis 2.0 Mol, vorzugsweise 0.5 bis 1.5 Mol an Reaktionshilfsmittel ein.

Die Reaktionsdurchführung, Aufarbeitung und Isolierung der Reaktionsprodukte erfolgt nach allgemein üblichen Methoden.

Als Katalysatoren zur Durchführung des erfindungsgemäßen Verfahrens (e) kommen alle üblichen Hydrierkatalysatoren in Frage. Vorzugsweise verwendet man Edelmetallkatalysatoren, wie beispielsweise Platin, Platinoxid, Palladium oder Ruthenium gegebenenfalls auf einem geeignetem Träger wie beispielsweise Kohlenstoff oder Siliciumdioxid.

Als Verdünnungsmittel zur Durchführung der erfindungsgemäßen Verfahrens (e) kommen inerte organische Lösungsmittel in Frage. Hierzu gehören insbesondere aliphatische oder alicyclische gegebenenfalls halogenierte Kohlenwasserstoffe, wie beispielsweise Benzin, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Alkohole wie Methanol, Ethanol oder Propanol.

Das erfindungsgemäße Verfahren (e) kann gegebenenfalls in Gegenwart eines geeigneten Säurebindemittels durchgeführt werden. Vorzugsweise verwendet man Alkalimetallcarbonate wie Natriumcarbonat oder Kaliumcarbonat oder organische Basen wie Pyridin oder Lutidin.

Die Reaktionstemperaturen können bei der Durchführung des erfindungsgemäßen Verfahrens (e) in einem größeren Bereich variiert werden. Im allgemeinen arbeitet man bei Temperaturen zwischen -20 °C und 100 °C, vorzugsweise bei Temperaturen zwischen 0 °C und 50 °C.

Das erfindungsgemäße Verfahren kann bei Normaldruck oder unter erhöhtem Druck durchgeführt werden. Vorzugsweise arbeitet man unter Normaldruck.

Zur Durchführung des erfindungsgemäßen Verfahrens (e) setzt man pro Mol an N-Aryl-Stickstoffheterocyclus der Formel (Ic) im allgemeinen 1.0 bis 3.0 Mol, vorzugsweise 1.0 bis 1.5 Mol an Wasserstoff und gegebenenfalls 1.0 bis 3.0 Mol, vorzugsweise 1.0 bis 1.5 Mol an Säurebindemittel ein.

Die Reaktionsdurchführung, Aufarbeitung und Isolierung der Reaktionsprodukte erfolgt nach allgemein üblichen Methoden.

Das erfindungsgemäße Verfahren (f) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsauretriamid.

Als Säureakzeptoren können bei dem erfindungsgemäßen Verfahren (f) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (f) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (f) wird im allgemeinen bei Normaldruck durchgeführt.

Zur Durchführung des erfindungsgemäßen Verfahrens (f) setzt man je Mol Hydroxyarylimid der Formel (IV) im allgemeinen zwischen 1 und 2 Mol, vorzugsweise zwischen 1,1 und 1,5 Mol, Alkylierungsmittel der Formel (V) ein.

Die Reaktionsdurchführung, Aufarbeitung und Isolierung der Reaktionsprodukte erfolgt nach allgemein üblichen Methoden.

Als Halogenierungsmittel zur Durchfürhung des erfindungsgemäßen Verfahrens (g) kommen die üblichen für die Halogenierung von aromatischen Verbindungen verwendbaren Halogenierungsmittel in Betracht. Vorzugsweise verwendet man elementare Halogene, wie Chlor oder Brom, oder Halogenverbindungen, wie Sulfurylchlorid.

Verfahren (g) wird gegebenenfalls unter Verwendung von Katalysatoren durchgeführt. Als solche kommen vorzugsweise saure bzw. elektrophile Halogenverbindungen, wie z. B. Hydrogenchlorid, Hydrogenbromid, Aluminiumchlorid, Aluminiumbromid, Eisen(III)-chlorid oder Eisen(III)-bromid in Betracht.

Das erfindungsgemäße Verfahren (g) wird vorzugsweise unter Verwendung von Verdünnungsmitteln durchgeführt. Es kommen vor allem diejenigen organischen Solventien in Betracht, die bereits oben für Verfahren (f) angegeben wurden, daneben jedoch auch Essigsäure und/oder Wasser.

Die Reaktionstemperaturen können bei dem erfindungsgemäßen Verfahren (g) in einem größeren Bereich variiert werden. Im allgemeinen arbeitet man bei Temperaturen zwischen 0 °C und 150 °C, vorzugsweise bei Temperaturen zwischen 20 °C und 120 °C.

Verfahren (g) wird im allgemeinen bei Normaldruck durchgeführt.

Zur Durchführung des erfindungsgemäßen Verfahrens (g) setzt man je Mol Ausgangsverbindung der Formel (Id) im allgemeinen zwischen 1 und 5 Mol, vorzugsweise zwischen 1 und 3 Mol, Halogenierungsmittel ein.

Die Reaktionsdurchführung, Aufarbeitung und Isolierung der Reaktionsprodukte erfolgt nach allgmein üblichen Methoden.

Das erfindungsgemäße Verfahren (h) wird vorzugsweise unter Verwendung von Verdünnungsmitteln

durchgeführt. Es kommen vor allem diejenigen organischen Solventien in Betracht, die bereits oben für Verfahren (f) angegeben wurden, in der zweiten Stufe außerdem vorzugsweise auch Alkohole, wie Methanol, Ethanol oder Isopropanol.

Das erfindungsgemäße Verfahren (h) wird vorzugsweise in Gegenwart eines Säureakzeptors durchgeführt. Es kommen als solche vorwiegend diejenigen Säurebindemittel in Be tracht, die bereits oben für Verfahren (f) angegeben wurden.

Die Reaktionstemperaturen können bei dem erfindungsgemäßen Verfahren (h) in einem größeren Bereich variiert werden. Im allgemeinen arbeitet man bei Temperaturen zwischen -20 °C und +150 °C, vorzugsweise bei Temperaturen zwischen 0 °C und 100 °C.

Das erfindungsgemäße Verfahren (h) wird im allgemeinen bei Normaldruck durchgeführt.

Zur Durchführung des erfindungsgemäßen Verfahrens (h) setzt man je Mol Arylamin der Formel (III) im allgemeinen zwischen 0,8 und 1,5 Mol, vorzugsweise zwischen 1,0 und 1,2 Mol, Chlorameisensäureester der Formel (VI) und je Mol Arylurethan der Formel (VII) im allgemeinen zwischen 0,8 und 1,5 Mol, vorzugsweise zwischen 1,0 und 1,2 Mol, Piperidin-2-carbonsäureester der Formel (VIII) ein.

Die Reaktionsdurchführung, Aufarbeitung und Isolierung der Reaktionsprodukte erfolgt nach allgemein üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selek tive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist je doch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen sowohl im Vorauflauf- als auch im Nachlauf-Verfahren.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in be stimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Tolu ol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl )-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphen oxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[-(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat

(TRIALLATE). Einige Mischungen zeigen überraschenderweise auch synergis tische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenflache, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele:

Beispiel 1:

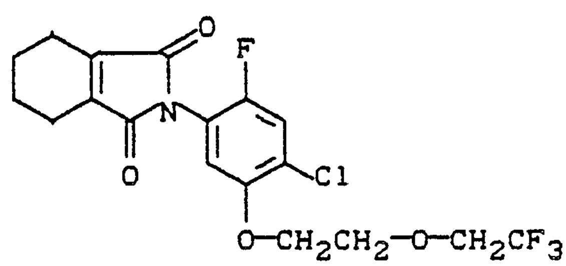

(Verfahren (a))

3,5 g (0,012 Mol) 4-Chlor-2-fluor-5-(2-(2,2,2-trifluorethoxy)-ethoxy)-anilin, 1,85 g (0,012 Mol) 3,4,5,6-tetrahydrophthalsäureanhydrid und 0,15 g (0,0012 Mol) 4-N,N-Dimethylaminopyridin werden in 20 ml Eisessig gelöst und 3,5 Stunden auf 90 °C erhitzt. Nach dem Abkühlen wird die Reaktionsmischung auf 100 ml Wasser ausgetragen und der entstandene Niederschlag mit Dichlormethan extrahiert. Die organische Phase wird nacheinander mit gesättigter Natrium-hydrogencarbonat-Lösung und gesättigter Natriumchlorid-Lösung gewaschen, über Magnesiumsulfat getrocknet und im Vakuum vom Lösungsmittel befreit. Als Rückstand erhält man 4,8 g (93,5 % der Theorie) 4-Chlor-2-fluor-5-(2(2,2,2-trifluorethoxy)-ethoxy)-1-(3,4,5,6-tetrahydrophtalimid)-phenyl vom Schmelzpunkt 45 °C bis 47 °C.

Analog Beispiel 1 und/oder entsprechend der allgemeinen Beschreibung der erfindungsgemäßen Herstellungsverfahren können die in der nachstehenden Tabelle 2 aufgeführten Verbindungen der Formel (I) erhalten werden.

Tabelle 2: Beispiele für die Verbindungen der Formel (I)

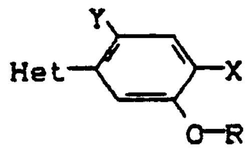

(I)

| Beisp.-Nr. | Het | X | Y | R | Physikal. Daten |
|---|---|---|---|---|---|
| 2 | 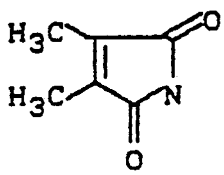 | Cl | F | $-CH_2CH_2-O-CH_2CF_3$ | Fp.: 75-77° C |
| 3 | 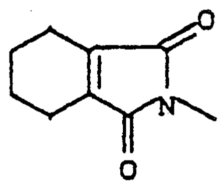 | Cl | F | 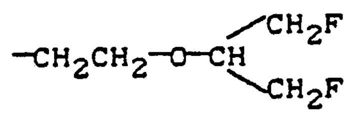 | 6,83[a)] |
| 4 | 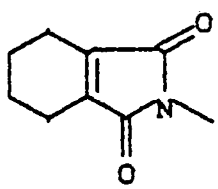 | Cl | F | 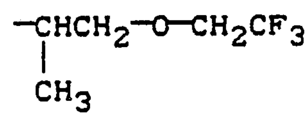 | 6,89[a)] |
| 5 | 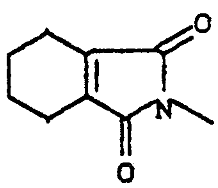 | Cl | F | 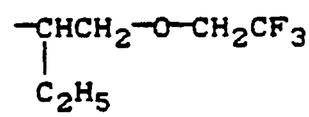 | 6,92[a)] |
| 6 | 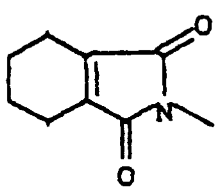 | Cl | F | 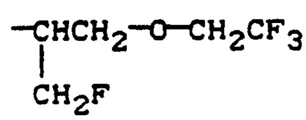 | 7,00[a)] |

Tabelle 2 - Fortsetzung

| Beisp.-Nr. | Het | X | Y | R | Physikal. Daten |
|---|---|---|---|---|---|
| 7 | 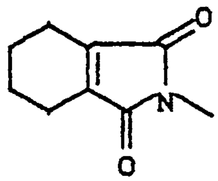 | Cl | F | $-CH_2CH_2OCH_2CH_2O-CH_2CF_3$ | 6,85[a)] |

a) $^1H$-NMR($CDCl_3$, $\delta$, ppm) : jeweils Dublett (J=6,5 Hz)

für 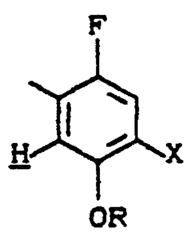

Ausgangsstoffe der Formel (III):

Beispiel (III-1):

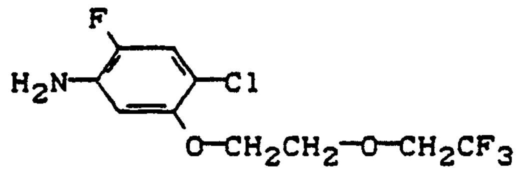

Zu einer Lösung von 5,5 g (0.034 Mol) 4-Chlor-2-fluor-5-hydroxyanilin in 80 ml N-Methylpyrrolidon gibt man 2,8 g (0,05 Mol) Kaliumhydroxid, gelöst in 3,5 ml Wasser. Es wird 15 Minuten gerührt und danach mit 7,0 g (0.034 Mol) 2-(2,2,2-trifluorethoxy)-ethylbromid versetzt. Das Reaktionsgemisch wird 16 Stunden bei Raumtemperatur gerührt und anschließend auf 200 ml Wasser ausgetragen.

Das ausfallende Öl wird mit Dichlormethan extrahiert, die organische Phase zweimal mit Wasser gewaschen, über Magnesiumsulfat getrocknet und im Vakuum vom Lösungsmittel befreit. Man erhält 8,2 g (84,4 % der Theorie) 4-Chlor-2-fluor-5-(2(2,2,2-trifluorethoxy)-ethoxy)-anilin als braunes Öl.

$^1$H-NMR($CDCl_3$, $\delta$) : 6,38 ppm (d, J = 8.03 Hz)

Analog können die in der nachstehenden Tabelle 3 aufgeführten Verbindungen der Formel (III) hergestellt werden.

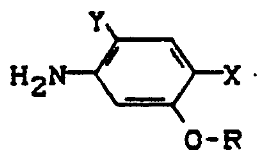

(III)

Tabelle 3: Ausgangsstoffe der Formel (III)

| Beip.-Nr. | X | Y | R | Physikal. Daten |
|---|---|---|---|---|
| III-2 | Cl | F | $-CH_2CH_2-O-CH_2CH_2-O-CH_2CF_3$ | 6,42[a] |
| III-3 | Cl | F | $CH_2F$<br>$-CH_2CH_2-O-CH$<br>$CH_2F$ | 6,40[a] |
| III-4 | Cl | F | $-CHCH_2-O-CH_2CF_3$<br>$C_2H_5$ | 6,49[a] |
| III-5 | Cl | F | $-CHCH_2-O-CH_2CF_3$<br>$CH_2F$ | 6,55[a] |
| III-6 | Cl | F | $-CHCH_2-O-CH_2CF_3$<br>$CH_3$ | 6,48[a] |

a) $^1H$-NMR($CDCl_3$, δ, ppm) : jeweils Dublett (J=8 Hz)

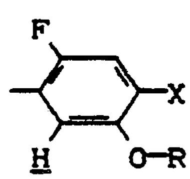

Ausgangsstoffe der Formel (V):

Beispiel (V-1):

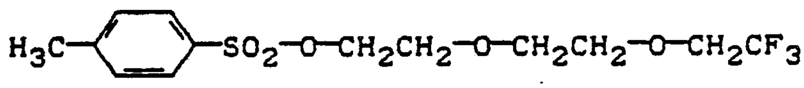

Zu einer Lösung aus 9,6 g (0.05 Mol) p-Toluolsulfochlorid und 9,5 g 2-(2-(2,2,2-trifluorethoxy)-ethoxy)-ethanol in 20 ml Dichlormethan gibt man bei 0° C bis 5° C 8,0 g (0,1 Mol) Pyridin. Es wird 3 Stunden bei Raumtemperatur nachgerührt und danach auf 40 g Eis und 15 ml konzentrierter Salzsäure ausgetragen. Die organische Phase wird abgetrennt, die wässrige Phase mit Dichlormethan extrahiert. Die vereinigten organischen Phasen werden zweimal mit Wasser gewaschen, über Magnesiumsulfat getrocknet und im Vakuum vom Lösungsmittel befreit. Als Rückstand erhält man 15,4 g (89 % der Theorie) 2-(2-(2,2,2-trifluorethoxy)ethoxy-)ethyl-p-toluolsulfonat als hellgelbes Öl.

$^1$H-NMR($CDCl_3$, $\delta$) : 7,35 ppm (d, J = 8.0 Hz)

Analog können die in der nachstehenden Tabelle 4 aufgeführten Verbindungen der Formel (V) hergestellt werden.

$X^1$-R (V)

Tabelle 4: Ausgangsstoffe der Formel (V)

| Beisp.-Nr. | $X^1$ | R | Physikal. Daten |
|---|---|---|---|
| V-2 | 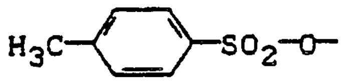 | 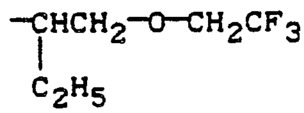 | 7,34[b)] |
| V-3 | 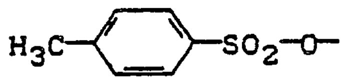 | 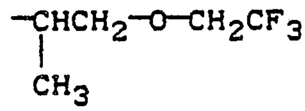 | 7,34[b)] |
| V-4 | 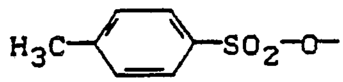 | 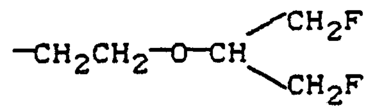 | 7,35[b)] |
| V-5 | 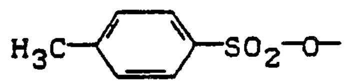 | 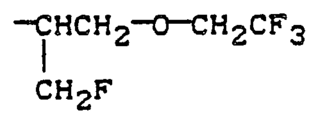 | 7,34[b)] |

b) $^1$H-NMR($CDCl_3$, δ, ppm) : jeweils Dublett (J=8 Hz)

für 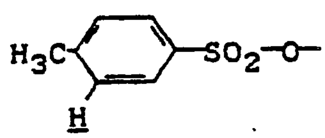

Beispiel (V-6):

$Br-CH_2CH_2-O-CH_2CF_3$

20 g (0,07 Mol) Phosphor(III)-bromid werden zu einer Mischung aus 28,8 g (0,2 Mol) 2-(2,2,2-Trifluorethoxy)-ethanol, 4 ml Pyridin und 150 ml Diethylether langsam unter Rühren tropfenweise gegeben. Das Reaktionsgemisch wird zunächst bis zum Ende der Zugabe bei 0 °C gerührt und dann 2 Stunden unter Rückfluß zum Sieden erhitzt. Anschließend wird vorsichtig mit 100 ml Eiswasser vermischt, nach kurzem

Nachrühren die organische Phase abgetrennt, mit 5%iger Natriumhydrogencarbonat-Lösung gewaschen, mit Natriumsulfat getrocknet und filtriert. Vom Filtrat wird das Lösungsmittel abdestilliert und der Rückstand bei Normaldruck destilliert.
Man erhält 20 g (48,5 % der Theorie) 2-(2,2,2-Trifluorethoxy)-ethylbromid vom Siedebereich 112 °C bis 116 °C (Brechungsindex: $n_D^{20}$ = 1,3828).

Herstellung von 2-(2-Fluor-1-fluormethyl-ethoxy)-ethanol:

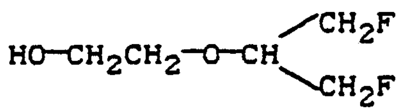

In einem V4A-Autoklaven (Volumen: 300 ml) werden bei -78 °C 112 g (1,0 Mol) 1,3-Difluor-2-propanol und 3 g Kaliumhydroxid vorgelegt und 50 ml Ethylenoxid einkondensiert. Dann wird das Reaktionsgemisch 12 Stunden bei +80 °C bis +90 °C (Anfangsdruck ca. 9000 hPa) geschüttelt. Nach Entspannen und Abblasen von nicht umgesetztem Ethylenoxid wird der restliche Autoklaveninhalt im Wasserstrahlvakuum fraktioniert destilliert. Man erhält 59 g (38 % der Theorie) 2-(2-Fluor-1-fluormethyl-ethoxy)-ethanol als Hauptfraktion vom Siedebereich 98 °C bis 105 °C / 24 hPa.
$^1$H-NMR($CDCl_3$, δ, ppm) : 2,75(s-breit, OH); 3,72(m, 2 x $CH_2$); 3,81(tq, CH); 4,52(dm, $2CH_2F$, $J_{HF}$ = 47,5 Hz).

Herstellung von 1-Fluormethyl-2-(2,2,2-trifluorethoxy)-ethanol:

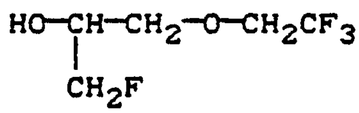

0,5 g Natrium werden bei 40 °C in 105 g (1,05 Mol) 2,2,2-Trifluorethanol gelöst und anschließend werden bei 50 °C bis 60 °C 76 g (1,0 Mol) Epifluorhydrin innerhalb von 30 Minuten tropfenweise dazugegeben. Das Reaktionsgemisch wird unter Rückflußkühlung 18 Stunden auf 70 °C erhitzt und dann im Wasserstrahlvakuum fraktioniert destilliert.
Man erhält 114 g (58 % der Theorie) 1-Fluormethyl-2-(2,2,2-trifluorethoxy)-ethanol als Hauptfraktion vom Siedebereich 70 °C bis 75 °C / 30 hPa.
$^1$H-NMR($CDCl_3$, δ, ppm) : 3,53(d, OH, $J_{HH}$ = 4,7 Hz); 3,71(m, $OCH_2$); 3,89(q, $CH_2CF_3$, $J_{HF}$ = 10,5 Hz); 4,03-(dm, CH); 4,46(dm, $CH_2F$, $J_{HF}$ = 46 Hz).

Herstellung von 1-Ethyl-2-(2,2,2-trifluorethoxy)-ethanol:

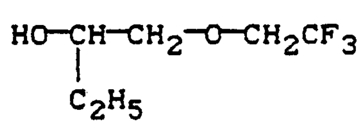

0,5 g Natrium werden bei 40 °C in 80 g (0,8 Mol) 2,2,2-Trifluorethanol gelöst und anschließend werden bei 50 °C bis 60 °C 43 g (0,6 Mol) Butylenoxid tropfenweise dazugegeben. Die Reaktionstemperatur wird langsam auf 110 °C gesteigert; der anfangs starke Rückfluß nimmt dabei rasch ab. Nach 17 Stunden wird im Wasserstrahlvakuum fraktioniert destilliert.
Man erhält 89 g (86 % der Theorie) 1-Ethyl-2-(2,2,2-trifluorethoxy)-ethanol als Hauptfraktion vom Siedebereich 52 °C bis 54 °C / 24 hPa.
$^1$H-NMR($CDCl_3$, δ, ppm) : 0,97(t, $CH_3$); 1,51(dq, $CH_2$); 2,75(d, OH, $J_{HH}$ = 4,8 Hz); 3,41 - 3,69(m, $CH_2O$); 3,74-

(m, CH); 3,88(q, $CH_2CF_3$, $J_{HF}$=9,9 Hz).

Analog erhält man 1-Methyl-2-(2,2,2-trifluorethoxy)-ethanol

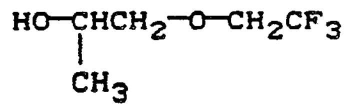

Siedebereich: 48° C bis 49° C / 24 hPa.

Anwendungsbeispiele

In den folgenden Anwendungsbeispielen wird die nachstehend aufgeführte Verbindung als Vergleichssubstanz herangezogen:

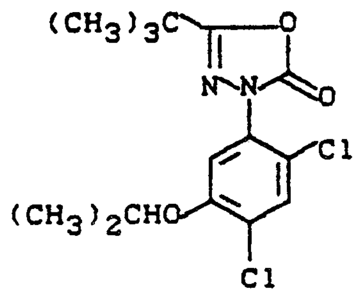

(A)

5-tert-Butyl-3-(2,4-dichlor-5-isopropoxy-phenyl)-1,3,4-oxadiazol-2-on (Oxadiazone/®Ronstar) - bekannt aus US-P 3 835 862.

Beispiel A

Pre-emergence-Test

Lösungsmittel: 5 Gewichtsteile Aceton

Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)

100 % = totale Vernichtung

In diesem Test zeigen beispielsweise die Verbindungen gemäß den Herstellungsbeispielen 1, 3, 4, 5, 6 und 7 bei voller Verträglichkeit gegenüber Weizen eine deutliche Überlegenheit in der Wirksamkeit gegen Unkräuter im Vergleich mit der bekannten Verbindung (A).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 1, 2, 3 und 7 bei guter Weizenverträglichkeit eine deutliche Überlegenheit in der Wirksamkeit gegen Unkräuter im Vergleich mit der bekannten Verbindung (A).

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert.

Eine deutliche Überlegenheit im Vergleich zur unbehandelten Kontrolle zeigen in diesem Test beispielsweise die Verbindungen gemäß den Herstellungsbeispielen: 1, 2, 3, 4, 5 und 7.

## Ansprüche

1. N-Aryl-Stickstoffheterocyclen mit fluorhaltigen Substituenten der Formel (I),

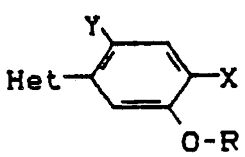

(I)

in welcher
Het für eine der nachstehenden heterocyclischen Gruppierungen

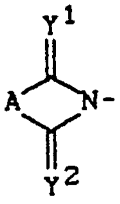

oder

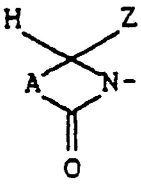

steht,

wobei
A für eine der nachstehenden Gruppierungen steht,

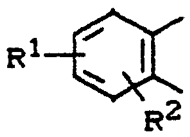 , 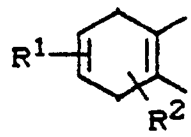 , 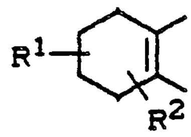 , 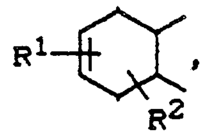 ,

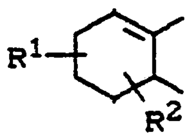 , 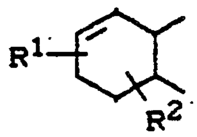 , 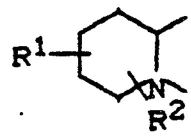 , 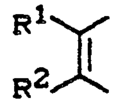 ,

wobei
$R^1$ und $R^2$ jeweils unabhängig voneinander für Wasserstoff, Halogen, Halogenalkyl oder Alkyl stehen,
$Y^1$ und $Y^2$ jeweils für Sauerstoff oder Schwefel stehen und
Z für Wasserstoff, Hydroxy oder Chlor steht,
R für jeweils gegebenenfalls verzweigtes, jeweils durch wenigstens ein Sauerstoffatom unterbrochenes und jeweils durch wenigstens ein Fluoratom substituiertes Alkyl, Alkenyl, Cycloalkyl, Cycloalkenyl, Cycloalkylalkyl oder Cycloalkenylalkyl steht,
X für Wasserstoff oder Halogen steht und
Y für Wasserstoff oder Halogen steht.

2. N-Aryl-Stickstoffheterocyclen mit fluorhaltigen Substituenten der Formel (I) gemäß Anspruch 1, in welcher
Het für eine der nachstehenden heterocyclischen Gruppierungen

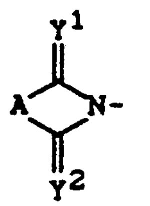 oder 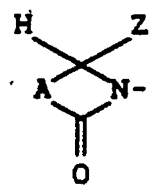

steht,
wobei
A für eine der nachstehenden Gruppierungen steht,

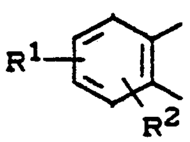 , 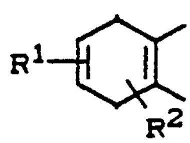 , 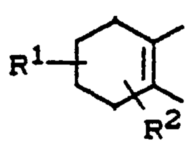 , 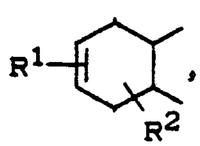 ,

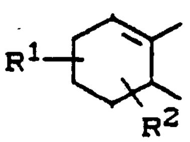 , 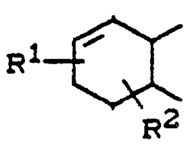 , 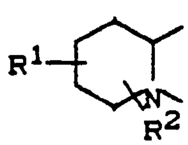 , 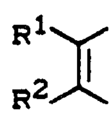 ,

wobei
$R^1$ und $R^2$ jeweils unabhängig voneinander für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit 1 bis 5 gleichen oder verschiedenen Halogenatomen stehen,
$Y^1$ und $Y^2$ für Sauerstoff oder Schwefel stehen,
Z für Wasserstoff, Hydroxy oder Chlor steht,
R für jeweils gegebenenfalls verzweigtes, jeweils durch 1 bis 4 Sauerstoffatome unterbrochenes und jeweils durch 1 bis 5 Fluoratome substituiertes Alkyl, Alkenyl, Cycloalkyl, Cycloalkenyl, Cycloalkylalkyl oder

Cycloalkenylalkyl mit jeweils bis zu 20 Kohlenstoffatomen steht,
X für Wasserstoff, Fluor, Chlor oder Brom steht und
Y für Wasserstoff, Fluor oder Chlor steht.

3. Verfahren zur Herstellung von N-Aryl-Stickstoffheterocyclen mit fluorhaltigen Substituenten der Formel (I)

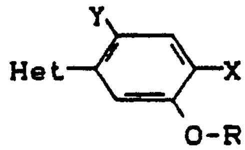 (I)

in welcher
Het für eine der nachstehenden heterocyclischen Gruppierungen

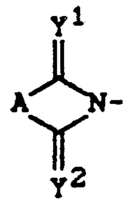 oder 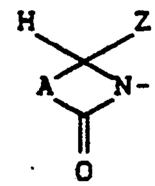

steht,
wobei
A für eine der nachstehenden Gruppierungen steht,

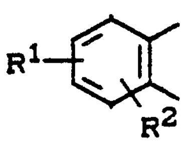 , 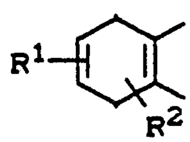 , 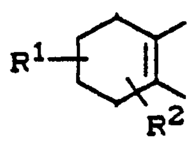 , 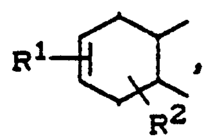 ,

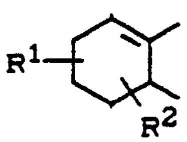 , 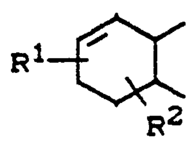 , 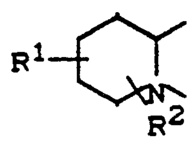 , 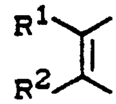 ,

wobei
$R^1$ und $R^2$ jeweils unabhängig voneinander für Wasserstoff, Halogen, Halogenalkyl oder Alkyl stehen,
$Y^1$ und $Y^2$ jeweils für Sauerstoff oder Schwefel stehen und
Z für Wasserstoff, Hydroxy oder Chlor steht,
R für jeweils gegebenenfalls verzweigtes, jeweils durch wenigstens ein Sauerstoffatom unterbrochenes und jeweils durch wenigstens ein Fluoratom substituiertes Alkyl, Alkenyl, Cycloalkyl, Cycloalkenyl, Cycloalkylalkyl oder Cycloalkenylalkyl steht,
X für Wasserstoff oder Halogen steht und
Y für Wasserstoff oder Halogen steht,
dadurch gekennzeichnet, daß man

(a) für den Fall, daß Het für die Gruppierung

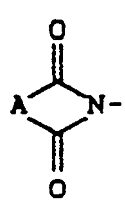

steht und
A, R, X und Y die oben angegebenen Bedeutungen haben,
cyclische Anhydride der allgemeinen Formel (II)

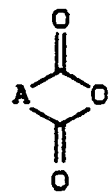

(II)

in welcher
A die oben angegebene Bedeutung hat,
mit Arylaminen der allgemeinen Formel (III)

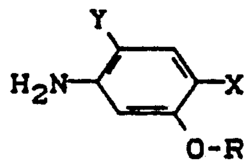

(III)

in welcher
R, X und Y die oben angegebenen Bedeutungen haben,
gegebenenfalls in Gegenwart eines Verdünnungsmittels und gegebenenfalls in Gegenwart eines Reaktionshilfsmittels umsetzt, oder daß man

(b) für den Fall, daß Het für die Gruppierung

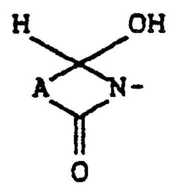

steht und
A, R, X und Y die oben angegebenen Bedeutungen haben,
substituierte Arylimide der allgemeinen Formel (Ia)

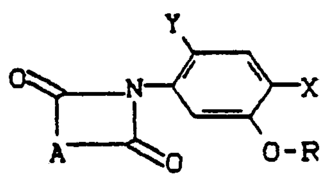

(Ia)

in welcher
A, R, X und Y die oben angegebenen Bedeutungen haben,
mit einem Reduktionsmittel, gegebenenfalls in Gegenwart eines Verdünnungsmittels umsetzt, oder daß man

(c) für den Fall, daß Het für die Gruppierung

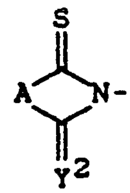

steht und
A, R, X, Y und $Y^2$ die oben angegebenen Bedeutungen haben,
substituierte Arylimide der allgemeinen Formel (Ia)

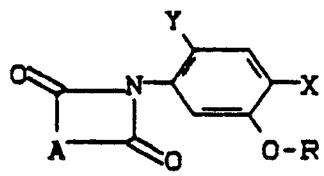

(Ia)

in welcher
A, R, X und Y die oben angegebenen Bedeutungen haben,
mit einem Schwefelungsmittel, gegebenenfalls in Gegenwart eines Verdünnungsmittel umsetzt, oder daß man

(d) für den Fall, daß Het für die Gruppierung

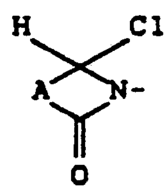

steht und
A, R, X und Y die oben angegebenen Bedeutungen haben,
N-Aryl-Stickstoffheterocyclen der allgemeinen Formel (Ib)

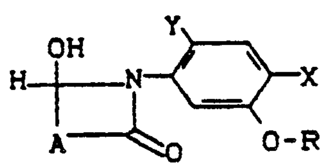

(Ib)

in welcher
A, R, X und Y die oben angegebenen Bedeutungen haben,
mit Thionylchlorid, gegebenenfalls in Gegenwart eines Verdünnungsmittels und gegebenenfalls in Gegenwart eines Reaktionshilfsmittels umsetzt, oder daß man

(e) für den Fall, daß Het für die Gruppierung

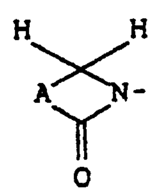

steht und
A, R, X und Y die oben angegebenen Bedeutungen haben,
N-Aryl-Stickstoffheterocyclen der allgemeinen Formel (Ic)

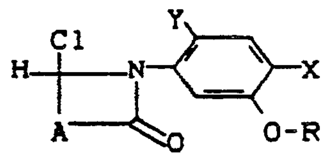

(Ic)

in welcher
A, R, X und Y die oben angegebenen Bedeutungen haben,
mit Wasserstoff, in Gegenwart eines Katalysators
sowie gegebenenfalls in Gegenwart eines Verdünnungsmittels und gegebenenfalls in Gegenwart eines Säurebindemittels umsetzt, oder daß man

(f) für den Fall, daß Het für die Gruppierung

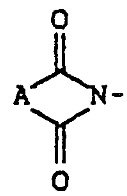

steht und
A, R, X und Y die oben angegebenen Bedeutungen haben,
Hydroxyarylimide der allgemeinen Formel (IV)

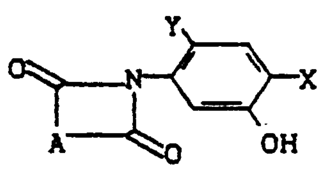

(IV)

in welcher
A, X und Y die oben angegebenen Bedeutungen haben,
mit Alkylierungsmitteln der allgemeinen Formel (V)

$X^1$ - R (V)

in welcher
R die oben angegebene Bedeutung hat und
$X^1$ für eine nucleophile Abgangsgruppe steht,
gegebenenfalls in Gegenwart eines Säureakzeptors und gegebenenfalls in Gegenwart eines Verdünnungsmittels umsetzt, oder daß man

(g) für den Fall, daß Het für die Gruppierung

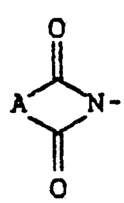

steht und
A, R und Y die oben angegebenen Bedeutungen haben sowie

X für Halogen steht,
substituierte Arylimide der allgemeinen Formel (Id)

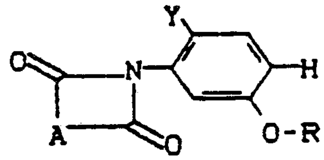
(Id)

in welcher
A, R und Y die oben angegebenen Bedeutungen haben,
mit einem Halogenierungsmittel, gegebenenfalls in Gegenwart eines Katalysators und gegebenenfalls in Gegenwart eines Verdünnungsmittels umsetzt, oder daß man

(h) für den Fall, daß Het für die Gruppierung

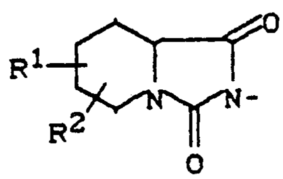

steht und
R, $R^1$, $R^2$, X und Y die oben angegebenen Bedeutungen haben.
Arylamine der allgemeinen Formel (III)

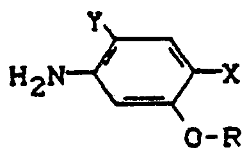
(III)

in welcher
R, X und Y die oben angegebenen Bedeutungen haben,
mit Chlorameisensäureestern der allgemeinen Formel (VI)
$R^3O$ - CO - Cl (VI)
in welcher
$R^3$ für $C_1$-$C_4$-Alkyl, Benzyl oder Phenyl steht,
gegebenenfalls in Gegenwart eines Säureakzeptors und gegebenenfalls in Gegenwart eines Verdünnungsmittels umsetzt und die hierbei gebildeten
Arylurethane der allgemeinen Formel (VII)

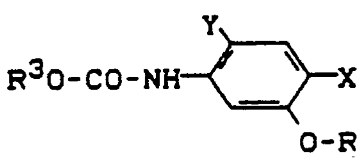
(VII)

in welcher
R, $R^3$, X und Y die oben angegebenen Bedeutungen haben,
mit Piperidin-2-carbonsäureestern der allgemeinen Formel (VIII)

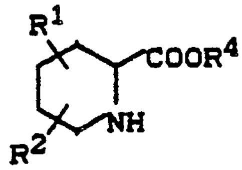

(VIII)

in welcher
$R^1$ und $R^2$ die oben angegebenen Bedeutungen haben und
$R^4$ für $C_1$-$C_4$-Alkyl steht,
gegebenenfalls in Gegenwart eines Verdünnungsmittels umsetzt.

4. Herbizide und pflanzenwuchsregulierende Mittel, gekennzeichnet durch einen Gehalt an mindestens einem N-Aryl-Stickstoffheterocyclus mit fluorhaltigen Substituenten der Formel (I) gemäß den Ansprüchen 1 bis 3.

5. Verfahren zur Bekämpfung von Unkräutern und zur Regulierung des Pflanzenwachstums, dadurch gekennzeichnet, daß man N-Aryl-Stickstoffheterocyclen mit fluorhaltigen Substituenten der Formel (I) gemäß den Ansprüchen 1 bis 3 auf die Unkräuter und/oder ihren Lebensraum einwirken läßt.

6. Verwendung von N-Aryl-Stickstoffheterocyclen mit fluorhaltigen Substituenten der Formel (I) gemäß den Ansprüchen 1 bis 3 zur Bekämpfung von Unkräutern und/oder als Pflanzenwachstumsregulatoren.

7. Verfahren zur Herstellung von herbiziden und/oder pflanzenwuchsregulierenden Mitteln, dadurch gekennzeichnet, daß man N-Aryl-Stickstoffheterocyclen mit fluorhaltigen Substituenten der Formel (I) gemäß den Ansprüchen 1 bis 3 mit Streckmitteln und/oder oberflächenaktiven Substanzen vermischt.

8. Arylamine der Formel (III)

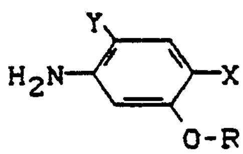

(III)

in welcher
R für jeweils gegebenenfalls verzweigtes, jeweils durch wenigstens ein Sauerstoffatom unterbrochenes und jeweils durch wenigstens ein Fluoratom substituiertes Alkyl, Alkenyl, Cycloalkyl, Cycloalkenyl, Cycloalkylalkyl oder Cycloalkenylalkyl steht,
X für Wasserstoff oder Halogen steht und
Y für Wasserstoff oder Halogen steht.

9. Alkylierungsmittel der Formel (V)

$X^1$-R (V)

in welcher
R für jeweils gegebenenfalls verzweigtes, jeweils durch wenigstens ein Sauerstoffatom unterbrochenes und jeweils durch wenigstens ein Fluoratom substituiertes Alkyl, Alkenyl, Cycloalkyl, Cycloalkenyl, Cycloalkylalkyl oder Cycloalkenylalkyl steht,
$X^1$ für eine nucleophile Abgangsgruppe, insbesondere für Chlor, Brom, Methylsulfonyloxy, Phenylsulfonyloxy oder Tolylsulfonyloxy steht.

10. Alkohole der Formel (XIVa)

HO-$R'$ (XIVa)

in welcher
$R'$ für verzweigtes und wenigstens durch 1 Sauerstoffatom unterbrochenes und wenigstens durch 1 Fluoratom substituiertes Alkyl steht.

11. Verfahren zur Herstellung von Arylaminen der Formel (III)

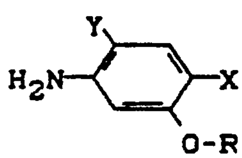

(III)

in welcher
R für jeweils gegebenenfalls verzweigtes, jeweils durch wenigstens ein Sauerstoffatom unterbrochenes und jeweils durch wenigstens ein Fluoratom substituiertes Alkyl, Alkenyl, Cycloalkyl, Cycloalkenyl, Cycloalkylalkyl oder Cycloalkenylalkyl steht,
X für Wasserstoff oder Halogen steht und
Y für Wasserstoff oder Halogen steht,
dadurch gekennzeichnet, das man
(α) Hydroxyarylamine der allgemeinen Formel (IX)

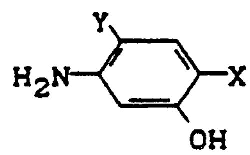

(IX)

in welcher
X und Y die oben angegebenen Bedeutungen haben,
mit Alkylierungsmitteln der allgemeinen Formel (V)
$X^1$ - R (V)
in welcher
R und $X^1$ die oben angegebenen Bedeutungen haben,
in Gegenwart eines Verdünnungsmittels, gegebenenfalls in Gegenwart eines Säureakzeptors sowie gegebenenfalls zusätzlich in Gegenwart von Wasser umsetzt, oder daß man
(β) Nitrophenol-Derivate der allgemeinen Formel (X)

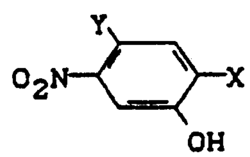

(X)

in welcher
X und Y die oben angegebenen Bedeutungen haben,
mit Alkylierungsmitteln der allgemeinen Formel (V)
$X^1$ - R (V)
in welcher
R und $X^1$ die oben angegebenen Bedeutungen haben,
nach der oben unter (α) angegebenen Methodik umsetzt und die so erhaltenen Verbindungen der allgemeinen Formel (XI)

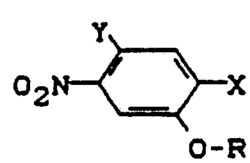

(XI)

in welcher
R, X und Y die oben angegebenen Bedeutungen haben, in Gegenwart eines Verdünnungsmittels reduziert, oder daß man
(γ) Phenol-Derivate der allgemeinen Formel (XII)

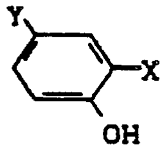

(XII)

in welcher
X und Y die oben angegebenen Bedeutungen haben,
mit Alkylierungsmitteln der allgemeinen Formel (V)

$X^1$ - R (V)

in welcher
R und $X^1$ die oben angegebenen Bedeutungen haben,
nach der oben unter (α) angegebenen Methode umsetzt und die so erhaltenen Verbindungen der Formel (XIII)

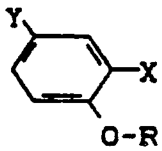

(XIII)

in welcher
R, X und Y die oben angegebenen Bedeutungen haben,
mit Salpetersäure zu den Verbindungen der Formel (XI) nitriert und anschließend reduziert.

12. Verfahren zur Herstellung von Alkylierungsmitteln der Formel (V)

$X^1$-R (V)

in welcher
R für jeweils gegebenenfalls verzweigtes, jeweils durch wenigstens ein Sauerstoffatom unterbrochenes und jeweils durch wenigstens ein Fluoratom substituiertes Alkyl, Alkenyl, Cycloalkyl, Cycloalkenyl, Cycloalkylalkyl oder Cycloalkenylalkyl steht,
$X^1$ für eine nucleophile Abgangsgruppe, insbesondere für Chlor, Brom, Methylsulfonyloxy, Phenylsulfonyloxy oder Tolylsulfonyloxy steht,
dadurch gekennzeichnet, daß man Alkohole der Formel (XIV)

HO-R (XIV)

in welcher
R die oben angegebene Bedeutung hat,

α) für den Fall, daß $X^1$ für Chlor oder Brom steht, mit einem Chlorierungsmittel oder mit einem Bromierungsmittel, gegebenenfalls in Gegenwart einer basischen Verbindung und gegebenenfalls in Gegenwart eines Verdünnungsmittels umsetzt oder

ß) für den Fall, daß $X^1$ für Methylsulfonyloxy, Phenylsulfonyloxy oder Tolylsulfonyloxy steht, mit Methansulfonsäurechlorid, Benzolsulfonsäurechlorid oder Toluolsulfonsäurechlorid, gegebenenfalls in Gegenwart einer basischen Verbindung und gegebenenfalls in Gegenwart eines Verdünnungsmittels umsetzt.